(19) Europäisches Patentamt
European Patent Office
Office européen des brevets

(11) **EP 3 432 940 B1**

(12) **EUROPEAN PATENT SPECIFICATION**

(45) Date of publication and mention
of the grant of the patent:
**05.11.2025 Bulletin 2025/45**

(21) Application number: **17716358.1**

(22) Date of filing: **27.03.2017**

(51) International Patent Classification (IPC):
**A61L 27/18** *(2006.01)*   **A61L 27/50** *(2006.01)*
**A61L 27/54** *(2006.01)*   **A61L 27/56** *(2006.01)*
**A61L 27/58** *(2006.01)*

(52) Cooperative Patent Classification (CPC):
(C-Sets available)
**A61L 27/18; A61L 27/50; A61L 27/54; A61L 27/56;**
**A61L 27/58;** A61F 2250/0023; A61F 2250/003;
A61L 2300/64; A61L 2430/10          (Cont.)

(86) International application number:
**PCT/US2017/024342**

(87) International publication number:
**WO 2017/165889 (28.09.2017 Gazette 2017/39)**

(54) **COMPLEX BRAIDED SCAFFOLDS FOR IMPROVED TISSUE REGENERATION**

KOMPLEXE FLECHTGERÜSTE ZUR VERBESSERTEN GEWEBEREGENERATION

ÉCHAFAUDAGES TRESSÉS COMPLEXES POUR UNE RÉGÉNÉRATION TISSULAIRE AMÉLIORÉE

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **25.03.2016 US 201662313246 P**

(43) Date of publication of application:
**30.01.2019 Bulletin 2019/05**

(73) Proprietor: **Biorez, Inc.**
**New Haven, CT 06513 (US)**

(72) Inventors:
• **ROCCO, Kevin, A.**
**New Haven, CT 06511 (US)**

• **PETERSON, Dale, R.**
**Carlsbad, CA 92009 (US)**
• **REILLY, Joseph, W.**
**Chatham, NJ 07928 (US)**

(74) Representative: **Carroll, Christopher P.**
**Boston & Galway**
**Golden Cross House**
**8 Duncannon Street**
**London WC2N 4JF (GB)**

(56) References cited:
**WO-A2-2004/080346      WO-A2-2009/109778**
**WO-A2-2011/119742      AU-B2- 2014 200 934**
**US-A1- 2014 172 096**

EP 3 432 940 B1

(52)  Cooperative Patent Classification (CPC): (Cont.)

C-Sets
**A61L 27/18, C08L 67/04**

## Description

### CROSS-REFERENCE TO RELATED APPLICATIONS

This application claims priority to U.S. Provisional Application No. 62/313,246 filed March 25, 2016.

### FIELD OF THE INVENTION

The present invention is in the field of implantable medical devices and prosthesis for rapid reconstruction, regeneration and replacement of tissue, including soft tissue, ligaments and tendons, with a complex braided structure tailored to produce desired mechanical properties and degradation profiles.

### BACKGROUND OF THE INVENTION

Injuries frequently occur in the musculoskeletal system, accounting for 60-67% of all unintentional injuries in the USA per annum (Ma et al., Nanomedicine, 8(9): 1459-1481 (2013)). It has been reported that more than 34 million musculoskeletal-related surgeries are performed each year in the USA (Deng et al, Trans Nanobiosci, 11L3-14 (2012)). Clinically, the main options available for the surgical treatment of musculoskeletal injuries include: transplantation of autografts/allografts/xenografts and utilization of synthetic substitutes composed of metals, ceramics, polymers and/or natural materials, such as silk, chitosan, or collagen. However, each strategy suffers from a number of limitations. For example, the benefits of auto grafts are counterbalanced by function loss and pain at the donor sites, scar tissue formation, structural differences between donor and recipient grafts preventing successful regeneration, and the shortage of graft material for extensive or additional repair. The use of allograft tissues obviates autograft donor-site complications, but can result in higher rates of failure, such as in younger and more active patients undergoing ACL reconstruction (Wassertein, Sports Health;7(3):207-216 (2015)). Allograft also varies in tissue quality based on donor, tissue source and processing method, and some patients and cultures remain averse to receiving cadaveric grafts (Pallis Am J Sports Med. Jun;40(6):1242-1246 (2012), Cheung Knee. 19(1):49-54 (2012)). Synthetic substitutes, such as metal prosthetics mainly serve as a replacement for damaged tissues or bone rather than as a platform for repair and regeneration of tissue defects. They are also often associated with issues such as poor integration with surrounding tissue and infection (Dale et al, Acta Orthop 83:449-458 (2012)).

To overcome the limitations associated with these approaches, regenerative medicine has emerged as a promising strategy for developing functional tissue constructs to reconstruct and restore damaged musculoskeletal tissues or organs (Badylak et al., Proc Natl Acad Sci USA, 107(8):3285-3286 (2010)). Three general strategies have been adopted for the creation of tissue constructs: to use isolated cells to produce tissues ex *vivo;* to use acellular biomaterials/scaffolds that are capable of inducing tissue regeneration *in vivo;* and to use a combination of cells and materials, typically in the form of scaffolds, for *in vivo* applications (Langer and Vacanti, Tissue engineering. Science, 260(5110):920-926 (1993); Khademhosseini et al., Proc Natl Acad Sci USA.; 103(8):2480-2487 (2006)). It is critical to design and fabricate a suitable scaffold for use in specific tissue regeneration, as it directly comes into contact with cells, and provides structural support and guidance for subsequent tissue development and regeneration (Khademhosseini et al., Progress in tissue engineering. Sci Am.; 300(5):64-71 (2009)). Towards this end, more and more attention has been paid to the design of scaffolds for guiding cell behaviors and tissue regeneration.

Significant advances in the design of fiber scaffolds for orthopedic and soft tissue repair and regeneration had been made in recent years. A number of polymeric scaffolds for repair and regeneration of soft tissues and musculoskeletal tissues are available for clinical use. These include STR GRAFT® (Soft Tissue Regeneration, Inc., CT, USA), SERICUFF (Serica, Allergan PLC, Irvine, CA, USA), BIOFIBER® (Tomier, Wright Medical, Memphis, TN, USA), LARS® Ligament (Dijon, France), FIX SORB® (Takiron Co., Ltd, Osaka, Japan; screws, nails, pins), NEOFIX® (Nicca USA Inc., CA, USA; screws, nails, pins), BIO-TENODESIS® interference screw (Arthrex, FL, USA), BIO-CORKSCREW® suture anchor (Arthrex), SMARTSCREW® (Conmed Linvatec, NY, USA), SMARTNAIL® (Conmed Linvatec), SMARTTACK® (Conmed Linvatec), SMARTPIN® (Conmed Linvatec), BIOSCREW® (Conmed Linvatec), BIOSTATAK® (Zimmer, IN, USA; suture anchor), prostatic stent, suture anchor, bone cement plug, BIOFIX® screws (BD Biosciences, NJ, USA), DEXON™ sutures and mesh (Covidien, Dublin, Ireland), BONDEK® suture (Teleflex, NC, USA), VALTRAC™ anastomosis ring and prostatic stent (Covidien), ARTELON® SPORTMESH™ (Artimplant, Västra Frölunda, Sweden), ARTELON® CMC Spacer Arthro (Artimplant), 3D OPLA® (open-cell polylactic acid) scaffold (BioMed Diagnostics, Inc., OR, USA), PHASIX™ Mesh (Davol Inc., RI, USA), ABSORB GT1 (Abbott, IL, USA) and many others. However, tailoring repair and regeneration scaffolds to provide the mechanical properties of orthopedic tissues, especially in the case where the material is biodegradable and ultimately replaced by endogenous cells has been difficult.

Synthetic ligament grafts or graft supports include carbon fibers, LEEDS-KEIO® ligament (polyethylene terephthalate), GORE TEX® prosthesis (polytetrafluoroethylene), STRYKER-DACRON® ligament prosthesis made of DACRON® tapes wrapped in a DACRON® sleeve and the Kennedy Ligament Augmentation Device (3M) made from polypropylene. These grafts exhibited good short term results but encountered clinical difficulties in long term studies. Limitations of these synthetic ligament grafts include long term laxity of the replacement material, weakened mechanical strength compared to the original structure and fragmentation of the replacement material due to wear. The United States Food and Drug Administration (FDA) does not approve or no longer approves these devices for sale, and there are no synthetic graft options currently available for anterior cruciate ligament reconstruction.

Natural ligaments are elongated bundles of collagenous soft tissue that serve, among other things, to hold the component bones of joints together. The desired characteristics for a ligament prosthesis include appropriate size and shape, biological compatibility, capability of being readily attached by the surgeon to the body of the patient, high fatigue resistance and mechanical behavior approximating that of the ligamentous tissue sought to be repaired or replaced.

Ligament constructs including collagen fibers, biodegradable polymers and composites thereof have been developed. Collagen scaffolds for ACL reconstruction seeded with fibroblasts from ACL and skin are described in Bellincampi, et al. J. Orthop. Res. 16:414-420 (1998) and PCT WO 95/2550. A bioengineered ligament model, which includes addition of ACL fibroblasts to the structure, the absence of cross-linking agents and the use of bone plugs to anchor the bioengineered tissue, has also been described (Goulet et al. Tendons and Ligaments. In R. P. Lanza, R. Langer, and W. L. Chick (eds), Principles of Tissue Engineering, pp. 639-645, R. G. Landes Company and Academic Press, Inc. 1997). U.S. Patent Application No. 20020123805 by Murray, et al. describes the use of a three-dimensional (three dimensional) scaffold composition which includes an inductive core made of collagen or other material, for repairing a ruptured anterior cruciate ligament (ACL) and a method for attaching the composition to the ruptured anterior cruciate ligament (See also U.S. Patent Application No. 20040059416). WO 2007/087353 discloses three-dimensional scaffolds for repairing torn or ruptured ligaments. The scaffold may be made of protein, and may be pretreated with a repair material such as a hydrogel or collagen. U.S. Patent Application No. 20080031923 by Murray, et al. describes preparation of a collagen gel and a collagen-MATRIGEL™ gel which is applied to a torn ligament for repair of the ligament. These collagen matrices are mostly monocomponent devices.

A number of multicomponent ligament prostheses have been described (U.S. Patent Nos. 3,797,047; 4,187,558; 4,483,023, 4,610,688 and 4,792,336). U.S. Patent No. 4,792,336 to Hlavacek, et al. discloses a device with an absorbable component comprising a glycolic or lactic acid ester linkage, and the remainder of the device comprising a non-absorbable component. The device includes a plurality of fibers comprising the absorbable component which can be used as a flat braid in the repair of a ligament or tendon. The required tensile strength is obtained by increasing the final braid denier. U.S. Patent No. 5,061,283 to Silvestrini discloses a bicomponent device comprising polyethylene terepthalate and a polyester/polyether block copolymer for use in ligament repair. U.S. Patent No. 5,263,984 to Li, et al, describes prosthetic ligament which is a composite of two densities of bioresorbable filaments.

Other polymeric scaffolds for articular tissue repair have been described in U.S. Patent Nos. 8,486,143 and 8,758,437, and U.S. Patent Application No. 20110238179 by Laurencin. These describe articular tissue repair devices containing polymeric three-dimensional braided scaffolds that degrade after a period of months. The scaffolds are formed from lactic acid polymers, are porous to allow cell ingrowth, but are slow to degrade and resorb *in vivo.*

WO 2009/109778 A2 discloses a device for replacement or regeneration of tendons and ligaments comprising a complex three-dimensional braided scaffold comprising polymeric multifilament yarn bundles, composite multifilament yarn bundles, composite yarn bundles, or combinations thereof, braided into a three-dimensional braided scaffold to form at least one end region for mechanical fixation of the device at a site of implantation and at least one tissue region for tendon or ligament tissue regeneration, and provides tissue function at the site of implantation as tissue integrates into the device, wherein the device comprises multifilament fibers, monofilament fibers, composite multifilament yarns, composite yarns, braided or twisted fibers, or any combination thereof, sewn, stitched, tied, or welded to the outside of the device and between the end region and the tissue region.

There is still a need for a device for reconstructing injured tissues, including ligaments and tendons, which mimics the strength and elasticity of the tissue, stabilizing the injured area, and encourages remodeling of soft tissue into a mechanically competent structure, and which can be easily implanted using existing surgical techniques.

It is an object of the present invention to provide biocompatible devices for reconstruction and regeneration of injured tissues that mimic the strength and elasticity of the tissues while stabilizing the injured area and permitting ingrowth of new tissue, and ultimately resorbing in a manner and duration consistent with and supportive of the regenerative and remodeling processes.

It is still another object of the present invention to provide methods for producing the devices for reconstruction and regeneration of injured tissues.

WO 2004/080346 A2 discloses a tissue-supporting prosthetic fabric for implantation. The fabric can carry functional groups, drugs and other biological reagents. The silk fibres are arranged in parallel and are optionally intertwined to form a construct. Sericin may be extracted at any point during the formation of the fabric, leaving a construct of silk fibroin fibres.

## SUMMARY OF THE INVENTION

The scope of the invention is defined by the claims. Any references in the description to methods of treatment refer to the products of the present invention for use in a method for treatment.

Devices for reconstruction and regeneration of injured tissues, which closely mimic the mechanical properties and elasticity of the tissues in need of reconstruction at the time of implantation, and methods of making and using the devices have been developed. The devices include complex three-dimensional braided scaffolds of biodegradable polymers having sufficient porosity for tissue ingrowth and regeneration. The scaffolds are typically braided with polymeric yarns of twisted/plied polymeric fibers using three-dimensional braiding and braiding designs to closely mimic the mechanical strength and elasticity of the tissue in need of reconstruction. The elasticity and modulus of these material structures prevents stress shielding and encourages the regeneration of functional tissue. The three-dimensional braided scaffolds can be braided into various shapes. The selection of shapes for the three-dimensional braided scaffolds can be guided by the anatomy of the tissue needing reconstruction. For example, reconstruction of ACL may require an elongated braided scaffold with length, width and cross-sectional area designed to match that of the native ACL. The degradation rate of the structure is designed to match the biologic regenerative process for a given tissue.

The end regions may include additional structures, such as sutures, and/or additional compositions and materials, such as porous metals, ceramics, polymers, or mineral/polymer composites to aid with mechanical fixation of devices to host tissues and/or encourage formation of a different tissue type such as bone.

The complex three-dimensional braided scaffolds have at least three levels of complexity: the fiber structure (monofilament fibers or multifilament fibers with varying filament number, diameter, denier, and polymer composition), the yarn structure (made of one or more types of fiber), and braiding design (made of one or more types of yarn structure) used to form the scaffolds.

The complex three-dimensional braided scaffolds are braided using polymeric yarn bundles. The yarn bundles are formed by twisting/plying multifilament fibers of the same or different structure, or a combination of multifilament fibers and monofilament fibers.

Typically, between about 10 and 100 polymeric filaments are extruded or combined to form multifilament fibers. The polymeric filaments have an average diameter ranging from between 1 micron and 50 microns and denier ranging from between 0.1 denier and 10 denier per filament (DPF). Any number, but typically between about 10 and 100, of multifilament fibers are then twisted/plied to form multifilament yarn bundles. Alternatively, the fibers are polymeric monofilament fibers, having an average filament diameter greater than 45 microns. Typically, between about 1 and 50 multifilament fibers and between about 1 and 50 monofilament fibers may be twisted/plied to form composite yarns.

The multifilament fibers used in the construct may be filaments of the same polymeric composition and diameter, the same polymeric composition but different diameter, different polymeric composition but the same filament diameter, or different polymeric composition and different filament diameter. The monofilament fibers may also vary in polymeric composition and diameter. Typically, the polymeric filaments and monofilament fibers are biodegradable polymers. The polymers can be homopolymers, copolymers in any configuration, such as random or block copolymers, polymer blends, or combinations thereof. Suitable polymers include, but are not limited to, biodegradable polyesters such as polylactide (PLA), polyglycolic acid (PGA), polycaprolactone (PCL), polydioxanone (PDO), the polyhydroxyalkanoates, such as poly(4-hydroxy butyrate), poly(3-hydroxy butyrate) and associated copolymers or blends thereof, polyanhydrides, poly(ortho esters), polyphosphazenes, poly(amino acids), polyalkylcyanoacrylates, poly(propylene fumarate), and trimethylene carbonate (TMC), as well as natural polymers such as silk, collagen, and chitosan, synthetically derived polymers of naturally occurring compounds, such as poly L-lactic acid (PLLA) and poly(glycerol sebacate), and nondegradable polymers such as polyester, polyethylene terephthalate (PET), polyethylene (PE), and acrylic polymers..

For example, about 48 multifilament poly(L-lactic acid) (PLLA) fibers, each fiber containing about 30 PLLA filaments, may be twisted/plied together to form a multifilament PLLA yarn bundle. In other embodiments, 24 multifilament fibers of one polymeric composition and 24 multifilament fibers of another polymeric composition, each fiber containing about 30 filaments, may be twisted together to form composite multifilament yarn bundles. In yet other embodiments, 1-24 monofilament fibers and 1-48 multifilament fibers, of the same or different composition, may be twisted/plied together to form composite yarn bundles.

The complex three-dimensional braided scaffolds may be braided using different braiding designs on a three-dimensional braider. The designs may include between 3 and 128 carriers carrying bobbins with yarn bundles. In one design having 36 carriers, the multifilament yarn bundles, composite multifilament yarn bundles, or composite yarn bundles may be loaded in any combination on the 36 braiding carriers 1-36. For example, 3 different yarns may be loaded on each of the 12 carriers, or 12 different types of yarn may be loaded on three carriers each. In some embodiments, the braiding designs include centers, for example between 1 and 20, or more centers, which are pulled into the structure between carriers and not braided. The centers contain fibers and/or yarns of any structure and composition. For example, the centers may be monofilament fibers, multifilament fibers, multifilament yarns, composite multifilament yarns, composite yarns, or any combination thereof. In other embodiments, the device may include between 0 and 50 multifilament fibers, monofilament fibers, multifilament yarns, composite multifilament yarns, composite yarns, braided or twisted fibers, or any combination thereof, sewn, stitched, tied, or welded throughout one or more regions of the device. The device may also include multifilament fibers, monofilament fibers, composite multifilament yarns, composite yarns, braided or twisted fibers, or any combination thereof, sewn, stitched, tied, or welded to, on, within, or between one or more regions of the device. In this embodiment, fibers/yarns are sewn, stitched, tied, or welded between the end region and the tissue region to separate the different regions of the device.

In some embodiments as disclosed but not specifically claimed herein, the device includes one or more braid inserts incorporated into the device. Typically, the one or more braid inserts are incorporated at the end regions of the three-dimensional braided scaffold.

In some embodiments as disclosed but not specifically claimed herein, at least a part of the device is embedded in a foam or sponge. In other embodiments, the entire device is embedded in a foam or sponge. The scaffolds of the devices present a support surface for attachment and ingrowth of tissue cells. The fiber organization of the scaffolds using combinations of fiber sizes and types offers control for each fiber type having a unique and defined purpose. For example, fast resorbing multifilament fibers with small filament diameters have increased surface area to volume ratio to maximize the number of cells attaching to the scaffold and accelerating the reconstruction and regeneration process, while slower resorbing fibers or yarns can provide longer structural integrity. Additionally, 3-D braiding allows certain fibers to be placed strategically in the corners of the structure, or the edge/face of the structure, or in the center of the structure, or in many combinations to control the key properties and characteristics of the device, such as the device shape and mechanical properties throughout the resorption profile. Placing various fiber types such as composite yarns or monofilaments on some or all carriers, or in some or all centers, enables control of the mechanical properties, particularly the stiffness of the structure if the individual fiber/yarn tensions are monitored and controlled.

The devices are at least partially biodegradable. Typically, the time course for biodegradation of the devices, or portions of the devices, is matched with the time course of regeneration of the tissue being reconstructed. It is the complex three-dimensional braided structure of the scaffold, which enables use of multiple types of fibers, such as fibers differing in composition or size, that allows control over the degradation profile of the device. The degradation profile includes changes in one or more of mechanical strength, elongation, elastic modulus. The device facilitates tissue regeneration throughout the degradation period.

The degradation of the device occurs in three phases during the regeneration of new tissue. In the first phase, or support phase, the graft contributes most of the structural support of the structure, allowing cells to infiltrate, proliferate, and propagate throughout the open porous scaffold. The elastic nature and relative low stiffness of the device creates mechanobiologic influences on the cells that facilitate the creation of extracellular matrix proteins and tissue. In the second phase, or transition phase, the graft reduces significantly in ultimate strength, transferring and increasing the load onto the developing functional tissue. In the third and final phase, or the degradation phase, the biodegradable scaffold loses all strength, and the new tissue bears all the mechanical loading of the structure. As polymer resorbs in mass and volume, the tissue remodels into mature tissue, reclaiming the space vacated by resorbing polymer.

The support phase occurs within first 0 to 6 months following surgery, marked by the scaffold losing at least 50% strength. The transition phase occurs within 1-12 months following surgery, marked by a reduction of initial strength by at least 90%. The degradation phase occurs between 3 and 18 months, such as between 3 and 12 months, or between 3 and 6 months, following surgery, marked by a reduction of mass by at least 50% of the initial mass value. The newly formed tissue is functional between 3 and 12 months following implantation surgery. At the time of implantation and at least within one month following implantation, the devices mimic the mechanical strength and elasticity of the tissue being reconstructed.

In the preferred embodiment, the device loses 50% initial ultimate tensile strength or "strength" by 3 months (completion of phase 1), 90% strength by 6 months (completion of phase 2), and 50% mass by 12 months (completion of phase 3) following surgery. In another embodiment, the device loses 50% strength by 4 months, 90% strength by 8 months, and 50% mass by 18 months.

In another embodiment, the support phase may last the first 3 months, the transition phase may occur over the next 3 months (6 months following surgery), and the degradation phase may last the next 9 months, occurring from 6-18 months following surgery. The newly formed tissue is fully functional by 9 months.

Methods of making the three-dimensional braided scaffolds, kits containing the devices, and methods of using the devices are also described.

**BRIEF DESCRIPTION OF THE DRAWINGS**

Figures 1A-1G are schematics of various embodiments for filament, fiber, and yarn organization.
Figures 2A-2Q are schematic diagrams of braiding designs on a four-track three-dimensional braiding machine with the various possible arrangements of fibers and yarns in carriers and centers.

Figure 3 is a perspective view of the multi-region ligament reconstruction device.

Figures 4A is a perspective view of the bone attachment end (end region) of the device in Figure 3. Figure 4B is a perspective view of the ligament tissue scaffold middle region (tissue region) of the device.

Figures 5A and 5B are prospective views of the implantation of the device to replace a torn ACL.

Figure 6 is a schematic diagram of a braid insert.

Figure 7 is a line graph showing mechanical properties (Load (Newton) over Extension (mm)) of two complex three-dimensional braided scaffolds, D3 and D5, at time zero (T=0) and after 4 weeks incubation in PBS (T=4).

## DETAILED DESCRIPTION OF THE INVENTION

### I. Definitions

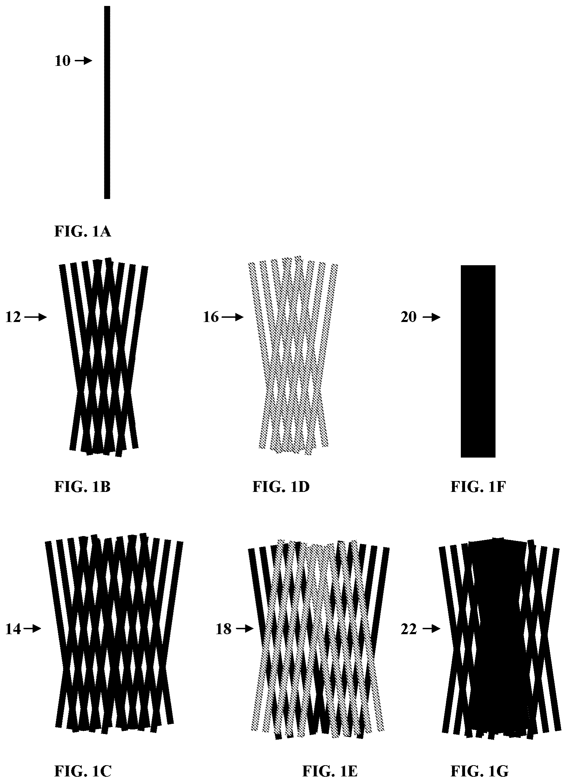

As used herein, the term "filament" refers to the simplest structural unit in a three-dimensional braided scaffold. See Figure 1A.

As used herein, the term "fiber" refers to a structural unit of a three-dimensional braided scaffold, wherein ten or more, typically between 10 and 100, filaments are twisted/plied together to form a multifilament fiber. Alternatively, "fiber" may be a monofilament fiber, in which case it is designated "monofilament fiber" or "monofilament fibers". See Figures 1B, 1D, and 1F.

As used herein, the term "yarn" refers to a structural unit of a three-dimensional braided scaffold, wherein monofilament fibers, multifilament fibers, or combinations thereof are twisted/plied together to form multifilament yarns (Figure 1C), composite multifilament yarns (Figure 1E), or composite yarns (Figure 1G), respectively. The multifilament yarns have multifilament fibers of same composition and diameter/denier. The composite multifilament yarns have multifilament fibers of different compositions and/or diameter/denier. The composite yarns have any combination of multifilament fibers and monofilament fibers of any composition. See Figure 1C, 1E, and 1G.

As used herein, the term "bundle" or "yarn bundle" refers to a bundle of fibers forming the yarn.

As used herein, the term "complex" when used in reference to three-dimensional braided scaffolds or three-dimensional braided structures refers to a scaffold or structure that has at least three levels of complexity: the fiber structure (monofilament fibers or multifilament fibers with varying filament number, diameter, denier, and polymer composition), the yarn structure (multifilament yarns, composite multifilament yarns, and composite yarns), and braiding design used to braid scaffolds or structures (number and arrangement of carriers and presence or absence of centers).

As used herein, the term "braid" refers to a three-dimensional braid braided using a three-dimensional braiding method and bundles of multifilament yarns, composite multifilament yarns, composite yarns, or combinations thereof.

As used herein, the term "braid insert" refers to a three dimensional object incorporated into the braided device such that fibers are braided around the braid insert.

As used herein, the term "degrade" refers to a reduction in one or more properties of the polymer forming fibers, yarns, or braided scaffolds, over time. The one or more properties are the molecular weight, total mass, mechanical strength, elasticity, or porosity of the fibers, yarns, or braided scaffolds. Biodegradable polymers can degrade enzymatically or through chemical hydrolysis of the backbone. In a bulk eroding polymer, the polymer network is fully hydrated and chemically degraded throughout the entire polymer volume. As the polymer degrades, the molecular weight decreases. The reduction in molecular weight is followed by a decrease in mechanical properties (e.g., strength) and scaffold properties. The decrease of mechanical properties is followed by loss of mechanical integrity and then erosion or mass loss (Pistner et al., Biomaterials, 14: 291-298 (1993)).

As used herein, the term "biocompatible" means the polymers forming the three-dimensional braided scaffolds do not typically generate systemic, toxic, acute, long-term (post-degradation of the device), and carcinogenic effects following implantation.

As used herein, the term "mechanical strength" refers to any one of ultimate tensile strength (maximum stress bared until failure (N)), peak load, load at yield, elongation at yield, tenacity, initial stiffness (N/mm), or the modulus of elasticity (Young's modulus). The modulus of elasticity measures an object or substance's resistance to being deformed elastically (i.e., non-permanently) when a force is applied to it. The elastic modulus of an object is defined as the slope of its stress-strain curve in the elastic deformation region. It can be measured using Formula (1):

$$E = Stress \: / \: Strain$$

where *Stress* is the force causing the deformation divided by the area to which the force is applied and *Strain* is the ratio of the change in some length parameter caused by the deformation to the original value of the length parameter. The modulus of elasticity is presented in Pascals (Pa), or megapascals (MPa).

As used herein, the term "porosity" or "void space" refers to the spacing or openings between filaments within each fiber, between fibers within each yarn, and between yarns within the scaffold, or the free volume fraction. The braided scaffold structure creates a large number of tiny filaments with a large surface area to volume ratio for maximum cellular attachment and proliferation (its surface area) while minimizing the space it occupies in the structure (its volume) to allow tissue to develop in the void spaces. The porosity is dependent on filament diameter, filament denier, fiber denier, fiber packing density, twisting/plying, braiding angle, and tension. The openings may be void spaces of any shape, including a spherical shape with a given diameter or a long, narrow, uninterrupted tunnel. Braiding creates void space between fibers that are not touching. For example, in the middle region of the device where the scaffold fibers are loosely braided (lower braiding angle, less picks per inch) there is a greater void space between the loose filaments. This void space is also uninterrupted void space having few constrictions less than 5 microns, which allows tissue to develop into an uninterrupted void space within the structure. In the preferred embodiment, more than 50% of the free volume in the scaffold has no constrictions smaller than 5 microns in the middle (tissue) region. The porosity is reported herein as a percent (%) volume fraction of a scaffold, or as a percent (%) volume fraction of a defined volume within a scaffold. A defined volume may be 1 $mm^3$, 5 $mm^3$, or 10 $mm^3$ segment of a scaffold.

As used herein, the term "support period" or "support phase" refers to a period of time following implantation of the device measured in days, weeks, or months, during which the scaffold of the device does not degrade, or minimally degrades, for example, retaining at least 50% of its initial ultimate tensile strength. The support period can be a period of time of 3 days, 7 days, 10 days, 15 days, 20 days, 25 days, 30 days (1 month), 1.5 months, 2 months, 2.5 months, 3 months, 4 months, 5 months, and 6 months. Some polymers lose a lot of strength in first few weeks (a burst loss) then more slowly resorb. Conversely some polymers lose strength slowly at first, then greatly accelerate

As used herein, the term "transition period" or "transition phase" refers to a period of time following the support period, measured in days, weeks, or months, during which the scaffold retains at least 10% of its initial ultimate tensile strength,. The transition period can be a period of time of 10 days, 15 days, 20 days, 25 days, 30 days (1 month), 1.5 months, 2 months, 2.5 months, 3 months, 4 months, 5 months, 6 months, 7 months, 8 months, 9 months or 10 months.

As used herein, the term "degradation period" or "degradation phase" refers to a period of time following the transition period, measured in days, weeks, or months, during which the scaffold degrades in mass by at least 50% of its initial mass. The degradation period can be a period of time of 10 days, 15 days, 20 days, 25 days, 30 days (1 month), 1.5 months, 2 months, 2.5 months, 3 months, 6 months, 12 months or 18 months.

Recitation of ranges of values herein are merely intended to serve as a shorthand method of referring individually to each separate value falling within the range, unless otherwise indicated herein, and each separate value is incorporated into the specification as if it were individually recited herein.

Use of the term "about" is intended to describe values either above or below the stated value in a range of approximately +/- 10%. The preceding ranges are intended to be made clear by context, and no further limitation is implied.

## II. Device

Implantable devices for reconstruction and regeneration of musculoskeletal tissues have been developed which at the time of implantation, and during an initial support period, mimic the structural and mechanical properties of and adopt the function of tissues in need of reconstruction and regeneration. The implanted devices may be fully or partly degradable over a defined period of time.

### A. Structure

The implantable devices include a complex three-dimensional braided scaffold. The scaffolds are braided from polymeric units as shown in Figures 1A-1G: filaments **10** form multifilament fibers **12.** Multifilament fibers **12** are twisted/plied together to form multifilament yarns **14.** The multifilament fibers **12** may be twisted/plied together with multifilament fibers **16** to form composite multifilament yarns **18.** In this embodiment, the multifilament fiber **16** differs from the multifilament fibers **12** in polymeric composition and/or denier.

Alternatively, monofilament fibers **20** and multifilament fibers, such as multifilament fibers **12** or **16,** may be twisted/plied together to form composite yarns **22.**

The scaffolds can have various dimensions and shapes, which match or closely mimic the shapes and mechanical properties of tissues in need of reconstruction or regeneration. For example, a device suitable for ACL reconstruction is an elongated device with a length ranging from between 85 and 130 mm, and diameter/width of between 2 mm and 16 mm, such as between 3 mm and 15 mm, 5mm and 10 mm, or 5 mm and 7 mm. The cross-sectional area, perpendicular to the main (longitudinal) axis of the scaffold, may be between 9 $mm^2$ and 650 $mm^2$, such as between 10 $mm^2$ and 600 $mm^2$, 20 $mm^2$ and 500 $mm^2$, 30 $mm^2$ and 400 $mm^2$, 50 $mm^2$ and 300 $mm^2$, or 100 $mm^2$ and 200 $mm^2$ The device may include a three-dimensional braided scaffold that is braided to include three regions: two end regions designed for mechanical fixation of the device at the site of implantation, which allow for bone cell ingrowth, and a middle region (tissue region), which serves as a scaffold for ligament or tendon cell ingrowth, resulting in the replacement of a ligament or tendon. The end regions of the device are used for mechanical fixation of the device to the bone in the bone tunnel zone. Typically, the middle region is long enough to enter the bone tunnel zone but is not used for fixing the device to the bone.

In this embodiment, the middle region differs from the two end-regions in one or more of fiber diameter, fiber structure, fiber twisting, fiber plying, yarn structure, yarn twisting, yarn plying, polymer composition, surface chemistry, braiding angle, porosity, void space volume, packing density, picks per inch, size, shape, tension, mechanical properties and degradation rate. Braiding can be defined in picks per inch which is how many twists occur along the braiding axis. For example, a loose braid can be formed by advancing the braider to increase the length while not braiding/twisting, thereby reducing picks per inch for that region, and creating a looser braid.

In some embodiments, the three-dimensional braided scaffolds may have end regions designed for attachment of the scaffold to host tissues. The end regions may include additional structures, such as sutures, and/or additional compositions and polymers, such as porous mineral/polymer composites to aid with attachment of devices to host tissues or to encourage formation of a different tissue type such as bone. End sections can also differ in chemical composition or contain cells, biologic fluids such as PRP, or natural or synthetic compounds such as proteins or growth factors to assist in formation of, and integration with, bone.

The device with a three-dimensional braided scaffold includes center fibers/yarn, for example between 1 and 50, or more center fibers/yarns, which are pulled into the scaffold but are not braided. The center fibers/yams are fibers and/or yarns of any structure and composition. For example, the center fibers/yams may be monofilament fibers, multifilament fibers, multifilament yarns, composite multifilament yarns, composite yarns, braided constructs, or any combination thereof. Typically, the center fibers/yams are under tension and end up mostly straight through the length of the scaffold between the braided fiber bundles. Although named "center fibers/yams", such fibers/yams may run through the scaffold at any off-center position along the length of the scaffold.

In other embodiments, the device may include between 0 and 50 multifilament fibers, monofilament fibers, multifilament yarns, composite multifilament yarns, composite yarns, braided or twisted fibers, or any combination thereof, sewn, stitched, tied, or welded throughout one or more regions of the device. For example, the device may include whipstitching (braided multifilament non-resorbable suture) on the end regions to aid in surgery/implantation The device may also include multifilament fibers, monofilament fibers, composite multifilament yarns, composite yarns, braided or twisted fibers, or any combination thereof, sewn, stitched, tied, or welded to the device and between the end region and the tissue region. In this embodiment, these fibers/yams are stitched and tied to separate the different regions of the device.

In some embodiments, the device includes one or more braid inserts incorporated into the device. Typically, the one or more braid inserts are incorporated at the end regions of the three-dimensional braided scaffold.

At least a part of the device may be embedded in a foam or sponge material. In some embodiments, the entire device is embedded in a foam or sponge material.

### 1. Filaments

Filament is the simplest structural unit in a three-dimensional braided scaffold. Filaments used in the formation of the three-dimensional braided scaffolds are polymeric structures of varying polymer composition, diameter, denier (D), and mechanical strength. The filaments may be formed of biodegradable, or slowly biodegradable polymers.

### a. Polymers

The polymeric filaments are typically biodegradable. The polymers suitable for forming the filaments may be homopolymers, copolymers, block copolymers, or blends. Biodegradable polymers include, but are not limited to, polyhydroxy acids such as polylactic and polyglycolic acids and copolymers thereof, polyanhydrides, polyorthoesters, polyphosphazenes, polycaprolactones, biodegradable polyurethanes, polyanhydride-co-imides, polypropylene fumarates, polydiaxonane, polyhydroxyalkanoates, poly(trimethylene carbonate), and/or combinations thereof in the form of blends and copolymers. Suitable polyhydroxyalkanoate homo polymers include poly-3-hydroxybutyrate (PHB), poly-4-hydroxybutyrate (P4HB), poly-3-hydroxyvalerate (PHV), poly-3-hydroxypropionate (PHP), poly-2-hydroxybutyrate (P2HB), poly-4-hydroxyvalerate (P4HV), poly-5-hydroxyvalerate (P5HV), poly-3-hydroxyhexanoate (PHH), poly-3-hydroxyoctanoate (PHO), poly-3-hydroxyphenylvaleric acid (PHPV) and poly-3-hydroxyphenylhexanoic acid (PHPH) can be used for self-retaining sutures. In alternative embodiments of the invention, polyhydroxyalkanoate copolymers including poly(3-hydroxybutyrate-co-3-hydroxyvalerate) (PHBV) and poly(3-hydroxybutyrate-co-3-hydroxyhexanoate) (PHBH).

Natural biodegradable polymers such as proteins and polysaccharides, for example, extracellular matrix components, collagen, fibrin, polysaccharide, a cellulose, silk, or chitosan, may also be used.

In some embodiments, the polymeric filaments include polymers that are flexible, elastic, or have low modulus of elasticity. Exemplary bioabsorbable polymers with low modulus of elasticity include polycaprolactone (PCL), poly(trimethylene carbonate) (PTMC), polydioxanone (PDO), poly(4-hydroxy butyrate) (PHB), and poly(butylene succinate) (PBS), and blends and copolymers thereof.

Scaffolds with a desired elastic modulus and degradation rate can be generated from filaments that are co-polymers incorporating co-monomers such as caprolactone, trimethylene carbonate, dioxanone, 4-hydroxyl butyrate, or butylene succinate. For example, the co-monomers may be lactide-caprolactone, lactide-trymethylene carbonate, lactide-4-hydroxy butyrate, or lactide-caprolactone-trymethylene carbonate, etc. Polymers may be combined as copolymers such as random, diblock or triblock copolymers with one or more block itself being a copolymer, while another block may have some other structure such as an alternating copolymer, block copolymer, random copolymer, or branch or pendant group. The co-monomer ratios in the co-polymers may range from between 100:0 and 0:100, or from between 100:0:0, 0:100:0, and 0:0:100, or from between 100:0:0:0, 0:100:0:0, 0:0:100:0, and 0:0:0:100. These ranges include any ratios falling within 100:0 and 0:100, such as ratios of between 30:70 and 70:30; within 100:0:0 and 0:0:100, or within 100:0:0:0 and 0:0:0:100.

For example, the PLA-PCL copolymers, or the PLA-PCL-PTMC co-polymers, may have co-monomer ratios as a weight fraction of the total weight of co-monomers, in any structural configuration, as presented in Table 1 below.

**Table 1. Ratios of co-monomers in a pre-mix of co-monomers in some embodiments of polymeric filament compositions.**

| Scaffold filament copolymer | Co-Monomers | Ratio |
|---|---|---|
| PLA-PCL | LA | 100:0 |
| | LA-CL | 95:5 |
| | LA-CL | 90:10 |
| | LA-CL | 85:15 |
| | CL | 0:100 |
| PLA-PCL-PTMC | LA | 100:0:0 |
| | LA-CL-TMC | 90:5:5 |
| | LA-CL-TMC | 80:10:5 |
| | LA-CL-TMC | 80:5:10 |
| | CL | 0:100:0 |
| | LA-CL-TMC | 70:15:15 |
| | LA-CL-TMC | 70:10:20 |
| | LA-CL-TMC | 70:5:25 |
| | TMC | 0:0:100 |

In other embodiments, the biodegradable polymers are polymers of lactic acid polymers such as poly(L-lactic acid (PLLA), poly(DL-lactic acid (PLA), and poly(DL-lactic-co-glycolic acid)(PLGA). The co-monomer (lactide-glycolide) ratios of the poly(DL-lactic-co-glycolic acid) are preferably between 100:0 and 50:50. Most preferably, the co-monomer ratios are between 85:15 (PLGA 85:15) and 50:50 (PLGA 50:50). Blends of PLLA with PLGA, preferably PLGA 85:15 and PLGA 50:50 can also be used.

In some embodiments, the polymeric filaments are formed of very slowly degrading polymers, which do not degrade during one to three years following implantation. Such very slowly degrading polymeric filaments may be used in yarns for forming the end sections of scaffolds and aid in attachment of the scaffolds to tissues. Polymers for very slowly degrading filaments include polyethylene, polystyrene, polyethylene terephthalate, silicone, polyfluoroethylene, polyacrylic acid, a polyamide (e.g., nylon, Kevlar), polycarbonate, polysulfone, polyurethane, polybutadiene, polybutylene, polyethersulfone, polyetherimide, polyphenylene oxide, polymethylpentene, polyvinylchloride, polyvinylidene chloride, polyphthalamide, polyphenylene sulfide, polyetheretherketone ("PEEK"), polyimide, polymethylmethacylate and/or polypropylene. In some cases, the polymer may include a ceramic such as tricalcium phosphate, hydroxyapatite, fluorapatite, aluminum oxide, or zirconium oxide, or porous mineral/polymer composites.

### b. Diameter

Filaments of various diameters may be used to form fibers. The filament diameter is between about 1 micron and 1000 microns. For example, the diameter of the filament may be about 1 micron, 5 micron, 15 micron, 25 micron, 35 micron, or 45 micron. Monofilament if used for structural support could exceed 35 micron, likely 150-200 micron or even larger such as 400-500 microns. The filament diameters within a multifilament fiber would all be less than 50 micron. These multifilament fibers enhance cell interactions.

### c. Denier

The filaments in multifilament fibers may have denier (D) ranging from between about 0.1 denier and 20 denier per filament (dpf). In preferred embodiments, the dpf ranges from between 1 and 5, more preferably from between about 2 and 3.

### d. Strength

The mechanical strength of the filaments is dependent on the filament diameter, draw ratio, and polymeric composition.

### 2. Fibers

There are at least three types of fibers. One type is a multifilament, formed by extrusion of multiple filaments having small diameter and low twist. A second type is a monofilament, a single filament, often having a diameter greater than 50 micron. A third type is a multifilament suture, which is similar to the multifilament but is separately braided into a tight construct. This multifilament suture may be heat set. The multifilament suture may behave as a single fiber. These can be used either in centers or twisted with the multifilaments into composite yarns and brought in centers or loaded onto bobbins, etc. Fibers formed from filaments are multifilament fibers. Two or more filaments may be twisted/plied together to form a multifilament fiber. Typically, between about 10 and 250 polymeric filaments are combined to form multifilament fibers. In preferred embodiments, the number of filaments per fiber is between about 20 and 60, more preferably about 30. See Figures 1A and 1B.

The multifilament fibers may be formed from filaments of the same polymeric composition and diameter, the same polymeric composition but different diameter, different polymeric compositions but the same filament diameter, or different polymeric composition and different filament diameter.

Alternatively, the fibers are monofilament fibers. The monofilament fibers may also vary in polymeric composition and diameter.

Typically, the polymeric filaments and monofilament fibers are biodegradable polymers. In some embodiments, the monofilament fibers may be bioabsorbable or non-bioabsorbable sutures. Examples of bioabsorbable sutures include sutures made from catgut (collagen), kangaroo tendons, glycolic acid polymers, l-lactic acid polymers, d-lactic acid polymers, trimethylene carbonate polymers, para-dioxanone polymers, epsilon-caprolactone polymers, polyhydroxyalkanoate polymers as well as copolymers using any combination of these materials as well as other chemically similar materials. Examples of non-bioabsorbable sutures include sutures made from polyamide, polybutylesters, polyetherester, polyetheretherketone, polyethylene, polyethylene terephthalate, polyurethane, polypropylene, polytetrafluoroethylene, metals, metal alloys, cotton and silk.

The classification of bioabsorbable and non-bioabsorbable sutures is not absolute. For example, most polyesters are non-bioabsorbable (such as polyethylene terephthalate) except that some polyesters (such as those made from polyglycolic acid, polylactic acid, or polyhydroxyalkanoates) are bioabsorbable. Similarly, silk is generally considered as a non-bioabsorbable material, but over a long period of time (e.g., 10 to 25 years), the body can break-down silk sutures implanted in the body, or the silk can be modified to be more bioabsorbable.

### a. Diameter

The diameter of the fibers is dependent on the number and the diameter of filaments used to form the fibers, as well as on the filament packing density, twisting/plying, and tension. For example, 20-60 microfilaments, each with a diameter of about 10-25 micron, may be used to form a multifilament fiber. The twisting of the filaments compacts the filaments so that the diameter of the fibers is typically about 50 to about 200 micron.

Monofilament fiber diameters are between about 20 microns and 700 microns. For example, the diameter of the monofilament fibers may be about 20 micron (USP 10-0), 110 micron, 120 micron, 130 micron, 140 micron, 150 micron (USP 4-0), 160 micron, 170 micron, 180 micron, 190 micron, 200 microns, or 600 micron.

**b. Denier**

Denier is a more useful measure of the fiber weight, and is dependent on the number of filaments per fiber, according to Formula I below:

$$FD = Nf^*Df$$

wherein *FD* is the fiber denier, *Nf* is the number of filaments in the fiber, and *Df* is the denier of one of the filament forming the fiber. Denier is typically expressed as the weight in grams of 9000 meters of fiber. A 9,000 meter length of 75 denier fiber weighs 75 grams.

For example, in a 30-filament PLLA fiber, wherein each PLLA filament has about 2.5 dpf, the fiber denier is 75 (30*2.5 = 75).

Typically, the denier of the multifilament fibers and monofilament fibers ranges from between 1 and 1000 denier.

**3. Yarns**

Any number of multifilament fibers, monofilament fibers, braided multifilament sutures, or combinations thereof may be twisted/plied together to form yarns. The yarns formed from multifilament fibers are multifilament yarns, the yarns formed from multifilament fibers of varying composition, diameter, and/or denier are composite multifilament yarns, and the yarns formed from multifilament and monofilament fibers are composite yarns (See Figures 1A-1G).

Any number, but typically between about 10 and 100 multifilament fibers are twisted/plied to form multifilament yarn bundles. In some embodiments, the multifilament yarns are formed from 10-60 multifilament fibers, each fiber containing between about 20 and 60 filaments, preferably about 30 filaments, and each filament having a diameter of 10-25 micron. In other embodiments, the composite yarns are formed from twisting between 1 and 50 multifilament fibers and between about 1 and 50 monofilament fibers, or combinations thereof, to form composite yarns.

**a. Denier**

The multifilament yarns, composite multifilament yarns, or composite yarns are between 100 and 64000 denier per yarn (dpy), preferably between about 1000 and 5000 dpy. Denier per yarn is dependent on the denier and the number of filaments in the fibers, and the number of fibers in the yarn, according to Formula II:

$$YD = NF^*Nf^*Df$$

wherein *YD* is yarn denier, or denier per yarn (dpy), *NF* is the number of fibers in the yarn, *Nf* is the number of filaments in the fiber, and *Df* is the denier of one of the filament forming the fiber.

For example, a PLLA yarn formed of 48 PLLA multifilament fibers, each fiber containing about 30 PLLA filaments, and each filament having about 2.5 denier, has a YD of 3600 dpy (48*30*2.5 = 3600).

**b. Strength**

The mechanical strength of the yarns is dependent on the polymeric composition of the fibers used, the draw ratio of the filaments, and heat treatments applied, and on the structural organization of the yarn. Typically, the yarn strength ranges from about 100 to 3000 MPa.

**4. Three-dimensional Braided Scaffolds**

The devices include complex three-dimensional braided scaffolds of biodegradable polymers. In some embodiments, the scaffolds may also include very slowly degrading polymers.

The scaffolds are typically braided with polymeric yarns using three-dimensional braiding technique and pre-selected braiding designs with carriers, or carriers and centers. The complex three-dimensional braided scaffolds have at least three levels of complexity: the fiber structure (monofilament fibers, multifilament fibers, or combinations thereof with varying filament number, diameter, denier, and polymer composition), the yarn structure (multifilament yarns, composite multifilament yarns, and composite yarns), and braiding design.

In some embodiments, the resulting braid can contain between 50% and 100% of multifilament fibers, such as between about 55% and 95%, 60% and 90%, 65% and 85%, 70% and 80%, 75%, 97.5%, or 99% of multifilament fibers, and between 50% and 0% of monofilament fibers, such as between about 45% and 5%, 40% and 10%, 35% and 15%, 30% and 20%, 25%, 2.5%, or 1% of monofilament fibers.

The braided scaffolds closely mimic the mechanical strength and elasticity of the tissue in need of reconstruction. It is the complex three-dimensional braided structure of the scaffold, which brings in multiple types of materials, in different sizes and configurations and allows control over the device characteristics, such as mechanical strength, elongation, elastic modulus, cell attachment and in-growth, and degradation/resorption profile. In addition, the complex braided structure allows packing of a large number of micron-diameter filaments (diameter of between 1 and 100 micron) into the volume of the device. This structure creates a large number of tiny filaments with a large surface area to volume ratio for maximum cellular attachment and proliferation (its surface area) while minimizing the space it occupies in the structure (its volume) to allow tissue to develop in the void space.

## a. Shape and Structure

The three-dimensional braided scaffolds can be braided into various shapes. The selection of shapes for the three-dimensional braided scaffolds can be guided by the anatomy of the tissue needing reconstruction.

The three-dimensional braided scaffolds can be uniform throughout the braided structure. In this embodiment, the scaffolds have uniform braiding angle, porosity, and degradation rate. The physical shape does not affect degradation. Scaffold has uniform braiding angle, picks per inch, braid packing and porosity.

In other embodiments, the three-dimensional braided scaffolds can have two or more regions that differ from each other in one or more of fiber diameter, fiber structure, fiber twisting, fiber plying, yarn structure, yarn twisting, yarn plying, polymer composition, surface chemistry, braiding angle, porosity, void space volume, packing density, size, shape, tension, mechanical properties and degradation rate. The three-dimensional braided scaffolds can also have end regions designed for attachment of the scaffold to host tissues. The end regions may further include other structures, such as sutures, braid inserts, and/or other biocompatible compositions and polymers, such as porous mineral/polymer composites to aid with attachment of devices to host tissues.

Therapeutic, prophylactic and/or diagnostic agents can be incorporated into the scaffold, as well as inorganic compounds. The agent can be encapsulated within the scaffold, dispersed within the polymer matrix that forms the scaffold, covalently or non-covalently associated with the surface of the scaffold or combinations thereof.

The agent to be encapsulated and delivered can be a small molecule agent (i.e., non-polymeric agent having a molecular weight less than 2,000, 1500, 1,000, 750, or 500 Dalton) or a macromolecule (e.g., an oligomer or polymer) such as proteins, enzymes, peptides, nucleic acids, etc. Suitable small molecule active agents include organic, inorganic, and/or organometallic compounds. The scaffolds can be used for *in vivo* and/or *in vitro* delivery of the agent.

Exemplary therapeutic agents that can be incorporated into the scaffolds include, but are not limited to, proteins, nucleic acid, saccharides and polysaccharides, and combinations thereof. These may be growth factors, anti-infectives, anti-inflammatories, local anesthetics, or hormones.

Exemplary diagnostic agents include paramagnetic molecules, fluorescent compounds, magnetic molecules, and radionuclides, x-ray imaging agents, and contrast agents.

Suitable biocompatible inorganic compositions include, for example, hydroxyapatite, alpha-tricalcium phosphate, beta-tricalcium phosphate, bioactive glass, calcium phosphate, calcium sulfate, calcium carbonate, xenogeneic and allogeneic bone material and combinations thereof. Suitable bioactive glass materials include silicates containing calcium phosphate glass, or calcium phosphate glass with varying amounts of solid particles added to control resorption time. Suitable inorganic compounds that may be incorporated into the calcium phosphate bioactive glass include, but are not limited to, magnesium oxide, sodium oxide, potassium oxide, and combinations thereof.

To achieve various degradation rates, the fiber can have the same polymeric composition and diameter, the same polymeric composition but different diameter, different polymeric compositions but the same filament diameter, or different polymeric composition and different filament diameter.

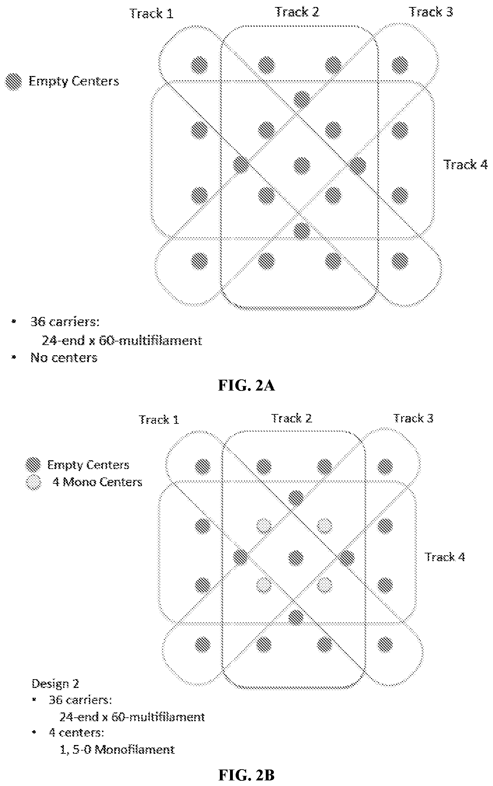

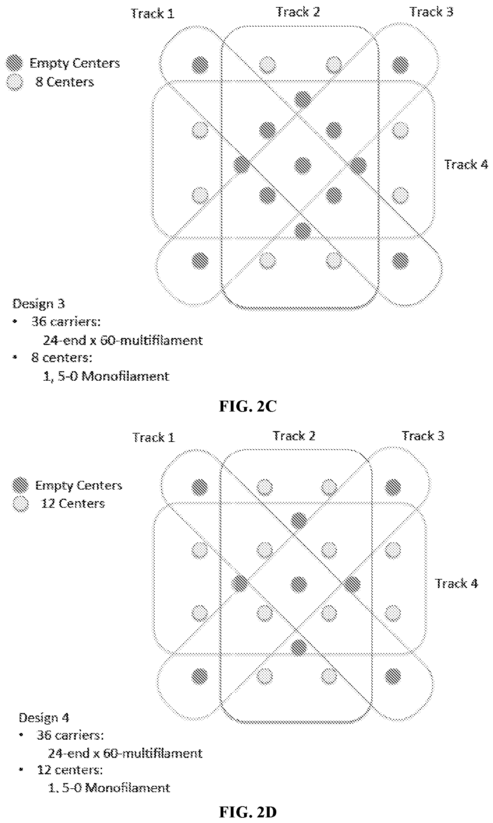

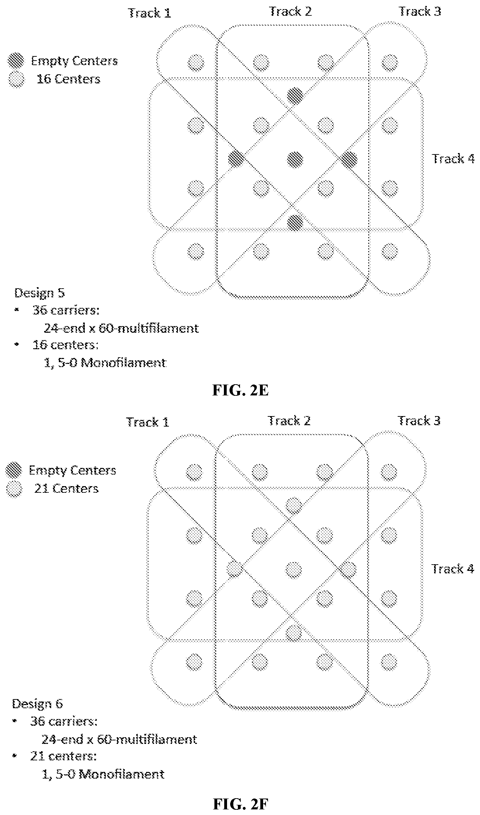

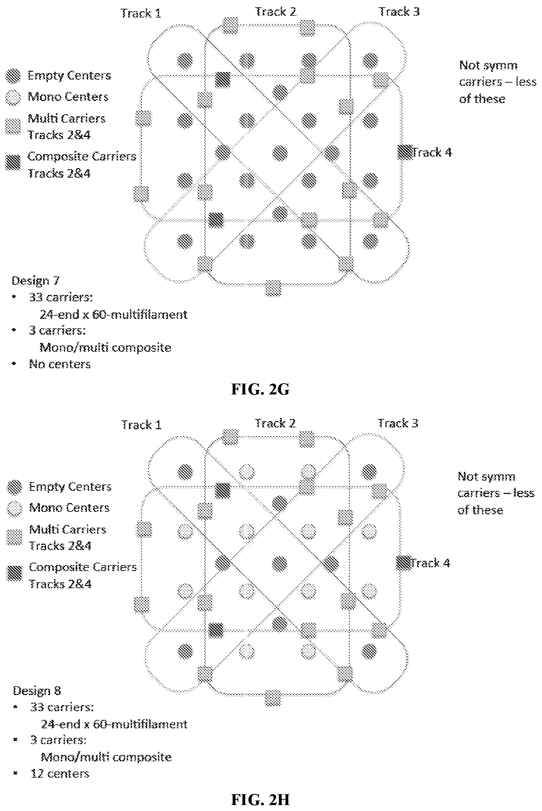

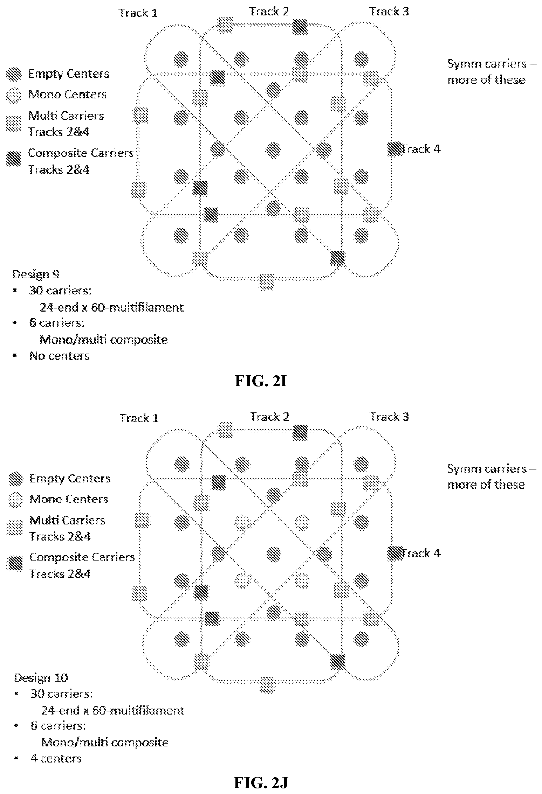

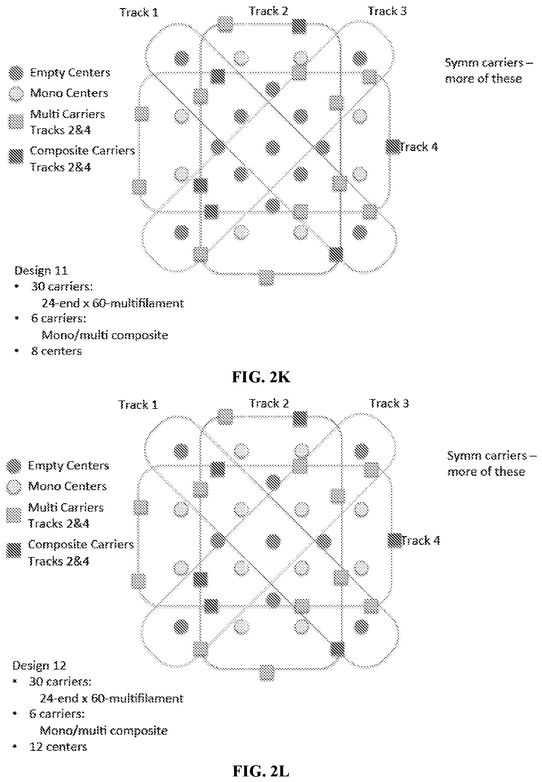

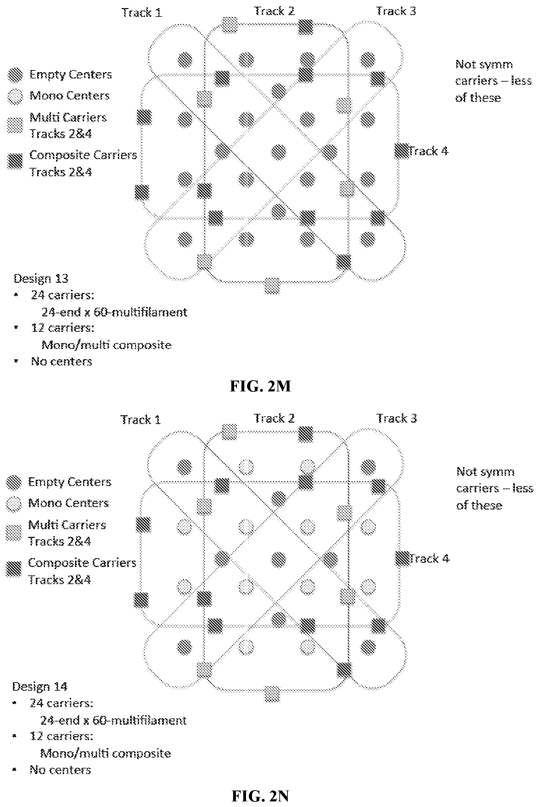

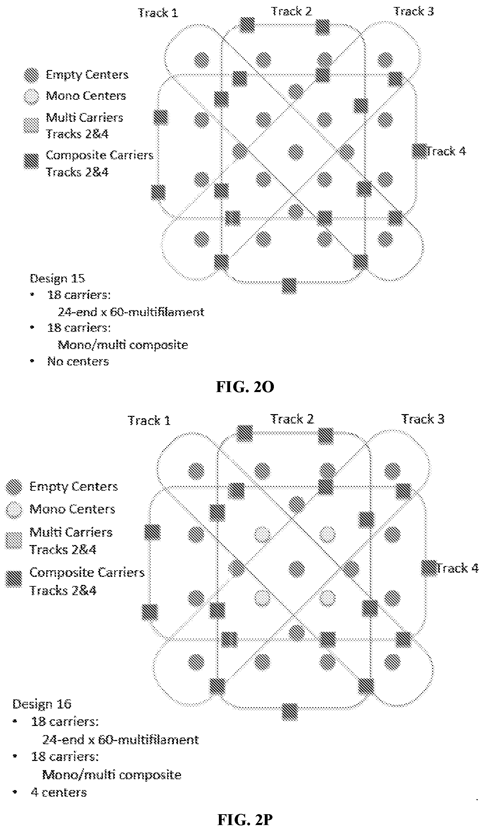

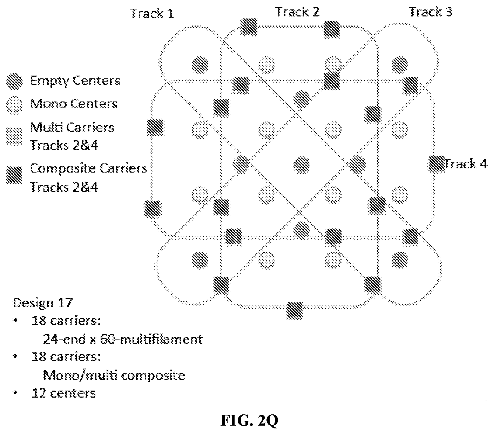

One or more fiber materials may be integrated into the braid via a 3-D carrier system that allows placement of certain fibers in certain parts of the braid. The complex three-dimensional braided scaffolds may be braided using different braiding designs on the three-dimensional braider (Figures 2A-2Q). The designs may include between 1 and 36 carriers carrying bobbins with yarn bundles. The multifilament yarn bundles, composite multifilament yarn bundles, or composite yarn bundles may be loaded onto any combination of the 36 braiding carriers 1-36. For example, 3 different yarns may be loaded on each of the 12 carriers, or 12 different types of yarn may be loaded on three carriers each. The centers contain fibers and/or yarns of any structure and composition. For example the centers may be monofilament fibers, multifilament fibers, multifilament yarns, composite multifilament yarns, composite yarns, or any combination thereof. The centers may pulled within the braid, but not braided, and tensioned separately from the braid. The carriers typically pass around these centers.

### b. Denier

The denier for the complex three-dimensional braided scaffolds is dependent on the scaffold structure and is calculated according to Formula III below:

$$BD = Ny * NF * Nf * Df$$

wherein *BD* is the braid denier (or denier per braid, dpb), *Ny* is the number of yarns in the braid, *NF* is the number of fibers in the yarn, *Nf* is the number of filaments in the fiber, and *Df* is the denier of one of the filament forming the fiber.

For example, a braided scaffold braided from 36 PLLA yarn bundles, each PLLA yarn bundle formed of 48 PLLA fibers, each fiber containing about 30 PLLA filaments, and each filament having about 2.5 denier, has a BD of 129,600 dpb (36*48*30*2.5 = 129,600).

Typically, the denier for the complex three-dimensional braided scaffolds ranges from between about 160 and 6,400,000.

### c. Porosity and Pore Size

Scaffolds typically possess a highly porous structure with an open fully interconnected geometry for providing a large surface area that will allow cell ingrowth, cell distribution, and, if needed, facilitate the neovascularization of the regenerating tissue. Average pore size, pore size distribution, pore volume, pore interconnectivity, pore shape, pore throat size, and pore wall roughness may vary with the individual scaffolds and are dependent on filament polymer composition, presence of coating materials, fiber and yarn architecture, and the braiding design. Typically, the scaffold provides a porous biocompatible network into which the surrounding tissue is induced and acts as a temporary template for the new tissue's growth and reorganization. Importantly, the fiber organization of the scaffolds offers an increased surface area to volume ratio to maximize the number of cells attaching to the scaffold and accelerating the reconstruction and regeneration process.

For example, 30 filaments of the same polymeric composition and the same diameter form a multifilament fiber, and 48 such fibers are twisted/plied together to create yarn bundles composed of 1440 filaments. The 1440 filaments are then braided on 36 carriers holding the yarn bundles to create a structure composed of 51,840 filaments. This structure creates a large number of tiny filaments with a large surface area to volume ratio for maximum cellular attachment and proliferation (its surface area) while minimizing the space it occupies in the structure (its volume) to allow tissue to develop in the void spaces.

The pore size and/or void space volume vary with individual scaffold design. The pore size, measured in any orientation, can have a diameter of 3 micrometers ($\mu$m, micron) or more for cellular ingrowth, 12 microns or more for neovascularization, and about 50 microns or more for bone ingrowth. The scaffolds can have pore openings with a diameter ranging from between about 5 $\mu$m and 20 $\mu$m, from about 20 $\mu$m and about 150 $\mu$m, from about 150 $\mu$m and about 350 $\mu$m, from about 200 $\mu$m and about 250 $\mu$m, or from about 100 $\mu$m and about 750 $\mu$m. The pores may be interconnected with much smaller void areas in order to provide for mass transfer of oxygen and nutrients.

The void space volume, or the porosity, may be as low as 10% to 20% within a 10 mm$^3$ segment of a scaffold, or as high as 50% to 90% within a 10 mm$^3$ segment of a scaffold. In some embodiments, the device includes an end region that has greater than 20% void space with no constriction smaller than about 3 microns, and a tissue region that has greater than 40% void space with no constriction smaller than about 3 microns.

### 5. Braid Insert

The device may include one or more braid inserts positioned at any location on the device. Preferably, the braid inserts are positioned at the end regions of the device, or at a transition from the middle region to the end region of the device.

### a. Shape and Dimensions

The braid insert is a three-dimensional object, such as a cube, a cuboid, a prism, a pyramid, a sphere, an oval, a cone, a dumbbell, or a cylinder.

The braid insert includes an axial through hole passing along the main axis of the braid insert. For example, when the braid insert is a cylinder, the axial through hole passes along the main, longitudinal, axis of the cylinder. In braid inserts without an identifiable main axis, such as braid inserts with a shape of a cube or a sphere, the main axis can be any axis passing through the braid insert.

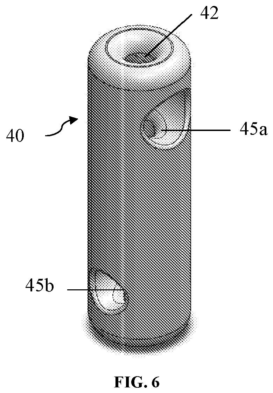

The braid insert may also include horizontal through holes, which are holes located on one or more axes offset from the main axis. An exemplary braid insert is presented in Figure 6. The braid insert **40** includes an axial through hole **42** and two horizontal through holes **45a** and **45b.**

The braid insert may have any suitable dimension that permits its incorporation into three-dimensional braid. The dimensions include a width, length, height, inner diameter and outer diameter, each of which may be between about 0.4 mm and 9 mm, such as 0.5 mm, 0.75 mm, 1 mm, 2 mm, 2.5 mm, 3 mm, 3.5 mm, 4, mm, 4.5 mm, 5 mm, 5.5 mm, 6 mm, 6.5 mm, 7 mm, 7.5 mm, 8 mm, 8.5 mm, 9 mm, 10 mm, 12.5 mm, 15 mm, 17.5 mm, 20 mm, 22.5 mm, and 25 mm. In some embodiments, the braid insert has an outer diameter between 0.5 mm and 5 mm, the inner diameter between 0.4 mm and 4.5 mm, and a length between 0.5 mm and 25 mm, such as length between about 1 mm and about 25 mm, about 2.5 mm and about 22 mm, about 5 mm and about 20 mm.

### b. Braid Insert Material

The braid insert is typically made from any bone compatible material, preferably a material that promotes and supports bone healing through osteogenic, osteoinductive, and/or osteoconductive mechanisms. Examples include polymers, metals, porous bone materials, allograft tissue, ceramic, minerals, natural materials, their blends, or combinations thereof. For example, the braid insert may be formed from titanium, biocompatible titanium alloys (e.g. $\gamma$Titanium Aluminides, Ti6-Al4-V ELI (ASTM F 136), or Ti6-Al4-V (ASTM F 1108 and ASTM F 1472)), stainless steel, cobalt-chrome, platinum, or any other biocompatible metal, or a combination thereof. Optionally, the metal is coated with a thermosetting polymer.

The braid insert may be formed of biocompatible polymers. Examples of polymers include biologically stable thermosetting polymers, such as polyethylene, polymethylmethacrylate, polyurethane, polysulfone, polyetherimide, polyimide, ultra-high molecular weight polyethylene (UHMWPE), cross-linked UHMWPE and members of the polyaryletherketone (PAEK) family, including polyether ether ketone (PEEK), carbon-reinforced PEEK, and polyether ketone ketone (PEKK). Preferred thermosetting polymers include, but are not limited to, polyether ketone ketone (PEKK) and polyether ether ketone (PEEK, e.g. PEEK-OPTIMA®, Invibio Inc). PEEK is particularly suitable because its modulus of elasticity closely matches that of bone.

The braid insert may be formed from ceramic material, such as hydroxyapatite, alpha-tricalcium phosphate, beta-tricalcium phosphate, biphasic calcium phosphate, calcium sulfate, synthetic bone, synthetic bone void fillers, or combinations thereof.

The braid insert may be formed from, or combined with, natural materials such as autograft, allograft, or xenograft, biocomposites, demineralized bone matrices (DBMs), bone marrow aspirate, platelet-rich plasma, blood, cells, or combinations thereof.

The braid insert itself may be radiopaque, or contain a radiopaque marker to facilitate visualization during imaging. The braid insert itself may contain sacrificial materials that dissolve or rapidly degrade, such as a salt crystals or degradable polymers, to create additional porosity in vivo, or to tune the overall degradation profile. The braid insert itself may contain additional cells, factors or proteins to promote bone formation or bone healing, such as bone morphogenic proteins, including BMP-2.

### c. Implantation

During device implantation, the braid inserts may be used as means to secure the device to the bone. For example, the horizontal through holes of the braid insert may accept fixation devices, such as commercially available fixation hardware, such as fixation screws, suspensory fixation tools, suture and whipstitching, ENDOBUTTON®, or other fixation equipment.

The braid insert helps prevent the collapsing of void space in the braid when the device is put under tension during implantation and during the patient gait cycle. The insert allows the device to maintain a larger outside diameter geometry, using a lower number of fibers than would otherwise be required. This enables more void space in the construct for tissue regeneration. For example, a cross-sectional diameter of 8 mm is typically achieved with 51,840 filaments, but by using an insert a similar cross-sectional diameter can be achieved using 25,920 filaments. This additional void space also improves embedding the scaffold into a composite material such as a collagen sponge.

The braid insert can be used as a bone composite with the scaffold, or can be trimmed away before implanting the device. The bone insert may be implanted with the scaffold to promote bone formation within the bone tunnels, which is dependent on the material choice and length of each region of the device.

The braid inserts can be resorbable, non-resorbing, very slowly resorbing, or combinations thereof, or may remain at the implantation site throughout the life of a subject receiving the device.

**6. Scaffolds for ACL Reconstruction**

Using ACL reconstruction as an example, an implantable device with a three-dimensional braided scaffold may be braided to have dimensions similar to those of the ACL. Suitable dimensions for a three-dimensional braided scaffold for ACL reconstruction include a length from between 85 and 140 mm, and diameter / width of between 5 and 15 mm. The cross-sectional area, perpendicular to the main axis of the scaffold, may range from between 30 mm$^2$ and 50 mm$^2$.

The scaffolds for ACL reconstruction can be braided to have at least three regions, two end regions designed for attachment of the device at the site of implantation, which allow for mechanical fixation and bone cell ingrowth, and a middle region which serves as a scaffold for ligament or tendon cell ingrowth, resulting in the replacement of a ligament or tendon. In this embodiment, the middle region differs from the two end-regions in one or more fiber diameter, fiber structure, fiber twisting, fiber plying, yarn structure, yarn twisting, yarn plying, polymer composition, surface chemistry, braiding angle, porosity, void space volume, packing density, size, shape, tension, mechanical properties and degradation rate. The middle region, therefore, represents the intra-articular zone of the ACL scaffold, and is more porous than the end regions (50% to at least 90% of the 10 mm$^3$ segment from this region of the scaffold is void volume). The end regions serve as bone tunnel zones for attachment of ACL scaffold to bones. The end regions are braided with a higher braiding angle than that for the middle region, and therefore have tighter braids with lower porosity.

Typically, the three-dimensional braided scaffolds for ACL reconstruction are braided to incorporate biodegradable polymeric yarns in the middle region, and very slowly degrading polymer filaments in the end regions. The end regions are used for mechanical fixation of the braid to the bone in the bone tunnel zone, and can contain additional materials, such as polyhydroxylethylmethacrylate (PHEMA), poly(methyl methacrylate) (PMMA), titanium, Ti-6Al-4V, CoCr, stainless steel, minerals or mineral-polymer composites such as bioglass, tricalcium phosphate, hydroxyapatite, or a combination of tricalcium phosphate and hydroxyapatite. The end regions can have pores with pores sizes of between 50 and 500 microns.

**a. Exemplary scaffold for ACL Reconstruction**

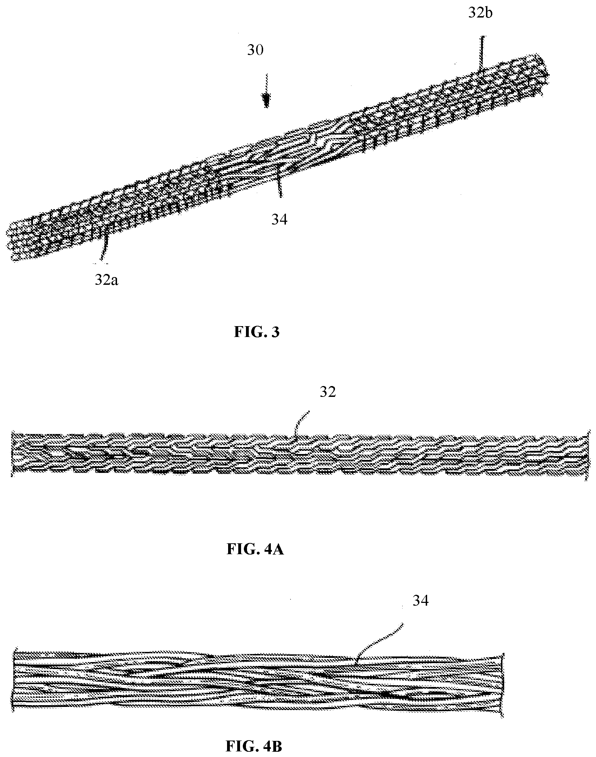

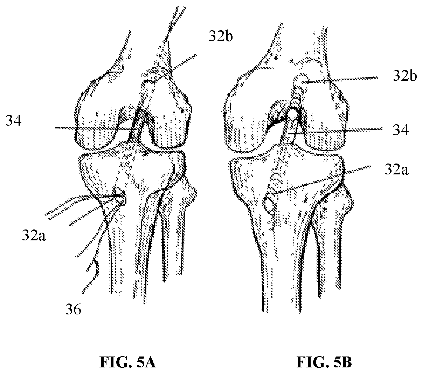

The preferred ligament or tendon reconstruction device **30** is shown in Figure 3, based on hierarchical design methodology. The device is made up of degradable polymeric fiber and one or more very slowly degradable polymeric fiber materials. In a preferred embodiment the device is composed of three regions, with two end regions **32a, 32b** designated for mechanical fixation of the device to the bone (shown in Figures 5A, 5B), and a middle region **34** which serves as the replacement intra-articular (ligament) tissue. In this embodiment, the middle region **34** differs from the two end regions **32a, 32b** in one or more of fiber diameter, fiber structure, polymer composition, braiding angle, porosity, size, and degradation rate (Figures 4A, 4B). The initial mechanical strength of the middle and end regions is preferably the same except for small differences caused by the extra braiding revolutions in the bony attachment region which slightly reduces strength. Ligament cell ingrowth occurs in the middle region and bone cell ingrowth occurs in the two end regions. Over time, the device begins to degrade. The middle region may degrade at one rate, or have areas of different rates of degradation.

The ends **32a, 32b** are placed in bone tunnels and secured with interference screws, rivets, sutures **36** or other techniques, as shown in Figures 5A and 5B. The device is intended to be implanted using the same techniques currently used by surgeons to implant patella tendon grafts.

**B. Stability and Degradation**

The biostability and biodegradation of the scaffolds depend on the chemical composition of the material and additional factors such as size, surface area to volume ratio, strength, elasticity, and contributions of the local environment such as mechanical forces, pH, exposure to water, cellular activity, mechanical wear etc.

Dependent on the polymeric composition, diameter and denier of the filaments, fibers, and yarns used for braiding the three-dimensional braided scaffolds, the scaffold can have varying stability and degradation rates. The stability of the scaffolds is characterized with the length of their support period, transition period, and degradation period. Some scaffolds may have a support period and degradation period only, each with various lengths, such as one to three months, after which the scaffolds is completely resorbed. Other scaffolds may have a support period, transition period, and degradation period, each with various lengths, such as one to three months or one to six months.

Biodegradation of polymeric biomaterials involves cleavage of hydrolytically or enzymatically sensitive bonds in the polymer leading to polymer chain breakage. The degradation rate of the scaffold is dependent on the degradation rate of the filaments, fibers, and yarns used to form the scaffolds.

For example, rapidly degrading polymeric filaments, fibers, and yarns lose between 80% and 90% of the polymer molecular weight within initial one week, two weeks, three weeks, one month, two months, three months following implantation. These filaments may be entirely degraded during the support period of the scaffold, leaving behind gradually degrading, slowly degrading, and very slowly degrading filaments, fibers, and yarns. Examples of such rapidly degrading filaments, fibers, and yarns include filaments, fibers, and yarns made from polyglycolic acid.

Gradually degrading polymeric filaments, fibers, and yarns lose between 5% and 10% of the molecular weight of the polymer within initial one week, two weeks, three weeks, one month, two months, three months following implantation. The polymeric filaments, fibers, and yarns then lose between 10% and 80% of their molecular weight within the next one month, two months, three months. These filaments may be almost entirely degraded during the transition period of the scaffold, leaving behind slowly degrading, and very slowly degrading filaments, fibers, and yarns.

Slowly degrading polymeric filaments, fibers, and yarns lose between 0% and 5% of the molecular weight within initial one week, two weeks, three weeks, one month, two months, three months following implantation. These filaments, fibers, and yarns then lose between 5% and 20% of their structural integrity within the next one month, two months, three months, and then fully degrade within the following one month, two months, three months. These filaments may be almost entirely degraded during the degradation period of the scaffold, leaving behind very slowly degrading filaments, fibers, and yarns.

Very slowly degrading filaments, fibers, and yarns remain stable during the initial one to three years of implantation, and may gradually degrade during next three to six years. Examples of such very slowly degrading filaments, fibers, and yarns include filaments, fibers, and yarns formed of polyethylene, polyether terephthalate, polystyrene, silicone, polytetrafluoroethylene, polyacrylic acid, a polyamide (e.g., nylon), polycarbonate, polysulfone, polyurethane, Hytrel, polybutadiene, polybutylene, polyethersulfone, polyetherimide, polyphenylene oxide, polymethylpentene, polyvinylchloride, polyvinylidene chloride, polyphthalamide, polyphenylene sulfide, polyetheretherketone ("PEEK"), polyimide, polymethylmethacylate and/or polypropylene. In some cases, the polymer may include a ceramic such as tricalcium phosphate, hydroxyapatite, fluorapatite, aluminum oxide, or zirconium oxide, bioglass, and additional polymers, such as polyhydroxylethylmethacrylate (PHEMA) and poly(methyl methacrylate) (PMMA).

Scaffold degradation can occur through mechanisms that involve physical or chemical processes and/or biological processes that are mediated by biological agents, such as enzymes in tissue remodeling. The biodegradable scaffold gradually degrades by predetermined period to be replaced by newly grown tissue from the adhered cells. Degradation results in scaffold dismantling and material dissolution/resorption through the scaffolds' bulk and/or surface types of degradation. Polymeric scaffolds undergoing bulk degradation have a breakdown of the internal structure of the scaffold thus reducing the molecular mass. A polymeric scaffold that primarily undergoes surface degradation can be described similarly to the dissolution of soap. The rate at which the surface degrades is usually constant. Therefore, even though the size of the scaffold becomes smaller, the bulk structure is maintained. These types of degrading scaffolds provide longer mechanical stability for the tissue to regenerate.

## C. Mechanical strength

The mechanical strengths of the three-dimensional braided scaffolds vary in accordance with the filaments' polymer composition and yarn braiding design. The scaffolds closely mimic the elastic modulus of the tissue in need of reconstruction.

The modulus of elasticity for a region of a scaffold disclosed herein may range from between 0.1 MPa and 20 GPa, and includes ranges of between about 0.1 MPa and 10 MPa, 10 MPa and 100 MPa, 100 MPa and 1 GPa, and 1 GPa and 20 GPa, when measured at room temperature and humidity.

The scaffolds typically have ultimate tensile strength and stiffness similar to the ultimate tensile strength and stiffness of the tissues in need of reconstruction. For example, scaffolds for ACL reconstruction may be generated to have a middle region with ultimate tensile strength at time zero (at time of implantation) ranges from between 500 N and greater than 4200 N, in some embodiments, between 1000 and 6500 N, and may be any value falling within these ranges. For example, suitable range for the ultimate tensile strength for an ACL scaffold's middle region may range from between 1700 N and 4000 N. Throughout the support period, the scaffold maintains greater than 80%, or greater than 90%, of its ultimate tensile strength.

The scaffolds may also vary in the stiffness, and may be designed to have an initial stiffness ranging from between 100 N/mm and 1000 N/mm. For example, scaffolds for ACL reconstruction may have a middle region with stiffness ranging from about 100 N/mm and 600 N/mm, while scaffolds for quadriceps and semitendinosus-gracilis graft may have a stiffness ranging from between 300 N/mm to 810 N/mm.

## D. Cell seeding

The devices can optionally be seeded with cells, preferably mammalian cells, more preferably human cells. Alternatively, they are implanted and cells may attach to and proliferate on and within the devices. Various cell types can be used for seeding. In a preferred embodiment, for ligament and tendon replacement, the cells are either mesenchymal in origin or capable of generating mesenchymal cells. Accordingly, preferred cell types are those of the connective tissue, as well as multipotent or pluripotent adult or embryonic stem cells, preferably pluripotent stem cells.

For regeneration or reconstruction of the ACL ligament, it may be preferable to seed the scaffold with ACL host cells, leaving stubs of the native ACL and implanting the device next to or into the stubs. However, the scaffolds can be seeded with any cell type which exhibits attachment and ingrowth and is suitable for the intended purpose of the braided scaffold. Some exemplary cell types which can be seeded into these scaffolds when used for reconstruction, regeneration or augmentation of connective tissue or other tissue types such as parenchymal tissues, include, but are not limited to, osteoblast and osteoblast-like cells, fibroblasts, chondrocytes, and progenitor cells such as myoblast or stem cells, particularly pluripotent stem cells.

Cells used may be harvested, grown and passaged in tissue cultures, they may be from a cultured cell line, or they may be from tissue harvested and dissociated at the time of implantation. In the preferred embodiment, cells are not seeded onto the scaffold but grow into the scaffold from the surrounding tissue. The cultured cells are then seeded onto the three dimensional braided scaffold to produce a graft material composed of living cells and partially degradable matrix. Each region of the scaffold can be seeded. Osteoblasts or mesenchymal stem cells in the end region help regenerate bone, ligament. Mesenchymal stem cells in the middle region regenerate the ligament. This graft material can then be surgically implanted into a patient at the site of ligament or tendon injury to promote healing and reconstruction of the damaged ligament or tendon.

In some embodiments, autologous bone marrow aspirates or a blood derivative such as platelet rich plasma can be used. The bone marrow aspirates contain platelet rich plasma (PRP) and bone marrow mononuclear cells (BM-MNC) and may accelerate tendon injury reconstruction. The bone marrow aspirates or platelet rich plasma may be applied together with the scaffold, or injected into the implantation site prior to or following scaffold implantation.

### E. Coatings

Synthetic materials, growth factors, proteins, and other bioactive agents may be used to coat fibers, yarns or scaffolds. Different regions of the device may be coated or embedded in different coating materials.

Examples of suitable synthetic coatings include hydrogels or cross-linking polymers such as polyethylene glycol and poly(glycerol sebacate), or osteoinductive, and osteoconductive materials.

Examples of suitable bioactive agents include the multitude of heterologous or autologous growth factors known to promote healing and/or regeneration of injured or damaged tissue. These growth factors can be incorporated directly into the scaffold, or alternatively, the scaffold can include a source of growth factors, such as for example, platelets. "Bioactive agents" include one or more of the following: chemotactic agents; therapeutic agents (e.g., antibiotics, steroidal and non-steroidal analgesics and antiinflammatories, anti-rejection agents such as immunosuppressants and anti-cancer drugs); various proteins (e.g., short term peptides, bone morphogenic proteins, glycoprotein and lipoprotein); cell attachment mediators; biologically active ligands; integrin binding sequence; ligands; various growth and/or differentiation agents and fragments thereof (e.g., epidermal growth factor (EGF), hepatocyte growth factor (HGF), IGF-I, IGF-II, TGF-p I-III, growth and differentiation factors, vascular endothelial growth factors (VEGF), fibroblast growth factors (FGF), platelet derived growth factors (PDGF), insulin derived growth factor (IGF) and transforming growth factors, parathyroid hormone, parathyroid hormone related peptide, beta-FGF; TGF-beta superfamily factors; BMP-2; BMP-4; BMP-6; BMP-12; sonic hedgehog: GDF5; GDF6; GDF8; MP52, CDMP1); small molecules that affect the upregulation of specific growth factors; tenascin-C; hyaluronic acid; chondroitin sulfate; fibronectin; decorin; thromboelastin; thrombin-derived peptides; heparin-binding domains; heparin; heparan sulfate; DNA fragments and DNA plasmids.

In a preferred embodiment, these include fibroblast growth factor (FGF), vascular endothelial growth factor (VEGF), epidermal growth factor (EGF), and bone morphogenic proteins (BMPs). Adhesive materials such as fibronectin and vimentin can also be used. These are preferably added in amount ranging from 0.1 nanogram to 1 micrograms.

### 1. Foam or Sponge

At least a part of the device may be embedded in a foam or sponge material. In some embodiments, the entire device is embedded in a foam or sponge material.

The pore size of the porous foam or sponge may be between about 5 $\mu$m and about 500 $\mu$m, between about 30 $\mu$m and 450 $\mu$m, between about 50 $\mu$m and 400 $\mu$m, between about 75 $\mu$m and 350 $\mu$m, between about 100 $\mu$m and 300 $\mu$m, and any value within these ranges. The pores are interconnected, allowing cells to enter the device and spread into the device before, during, or following implantation.

The foam or sponge may contact the device on the outside, fill in the void spaces of the device, or both. The foam or sponge may be cross-linked or be cross-linked to the fibers of the device. In some embodiments, the device is coated and embedded in the foam or sponge without crosslinking. The porosity of the foam or sponge increases the surface area to volume ratio of the device. The foam or sponge may be used with scaffolds having a lower volume of filaments without compromising the surface area of the scaffolds, which allows cellular attachment and proliferation.

For example, a three-dimensional braided scaffold without a foam or a sponge and braided with 51,840 filaments may have about the same total surface area as a three-dimensional braided scaffold with a foam or a sponge and braided with 17,280 identical filaments; due to the lower volume of sponge material needed to create an equivalent surface area. The later device's overall surface area to volume ratio is significantly higher compared to that of the former and allows for improved cellular attachment and proliferation.

The sponge or foam can have a degradation or resorption profile different than that of the filaments, typically faster than the filaments, creating a two or more stage degradation profile to improve healing and load transfer.

The addition of a foam or sponge can be used to hydrate the device with fluid prior to implantation. Exemplary fluids include blood, platelet rich plasma (PRP), bone-marrow derived mononuclear cells, bone marrow aspirate, or any preparation of patient fluid or cells. Alternatively, cells or fluids can be delivered or injected into the sponge following implantation or surgical fixation. The porous foam or sponge may be formed from any suitable material, such as synthetic or natural polymers, biologics, or a combination of polymers and biologics. Typically, the porous open-cell sponge is created by casting the braided scaffold into a solution and then lyophilizing the constructs. For example, a solution of polymer or collagen that is 5% by weight can be cast into a device mold and then lyophilized. This typically creates an open sponge with approximately 95% void space. This type of braided scaffold embedded within a filler, foam, or sponge, can be considered a composite material.

### a. Polymers

Suitable polymers include bioresorbable synthetic polymers, such as polyethylene glycol (PEG), poly(glycolic acid) (PGA), poly(lactic acid) (PLA), polydioxanone (PDO), polyglycerol polyricinoleate (PGPR), polymers of lactic acid and glycolic acid, polyanhydrides, poly(glycerol sebacate) (PGS), poly(ortho)esters, polyurethanes, poly(butic acid), poly(valeric acid), poly(caprolactone) (PCL), poly(hydroxybutyrate), poly(lactide-co-glycolide), poly(lactide-co-caprolactone), and copolmyers, blends, and chemical derivatives thereof (substitutions, additions of chemical groups, for example, alkyl, alkylene, hydroxylations, oxidations, and other modifications routinely made by those skilled in the art).

### b. Biologies

Biologics include peptides, proteins, proteoglycans, polysaccharides, phospholipids, sphingolipids, small molecules, bioactives, and combinations thereof.

Suitable proteins include lyophilized extracellular matrix proteins (ECM, including collagen, elastin, fibronectin, and laminin), serum proteins (such as albumin and immunoglobulins), growth factors, cytokines, and chemokines. Suitable proteoglycans include heparan sulfate, chondroitin sulfate, and keratan sulfate.

Suitable polysaccharides include hyaluronic acid, alginate, chitosan, dextran and cellulose, Suitable small molecules include cholesterol, vitamins, sugars, and amino acids. Suitable bioactives include therapeutic agents, such as anti-microbial agents.

### i. Collagen

Collagen may be in the form of procollagen, or as fibrillar (Type I, II, III, V, XI), FACIT (Fibril Associated Collagens with Interrupted Triple Helices, Type IX, XII, XIV), short chain (Type VIII, X), basement membrane (Type IV), other types, (Type VI, VII, XIII), or combinations thereof.

Collagen may be obtained from any animal source, such as human, bovine, ovine, equine, avian, or pig, or may be a synthetic collagen. Collagen may be obtained from any suitable organ, such as from skin, tendon, cartilage, bone, basement membrane, hair, or placenta.

Collagen may be lyophilized collagen, collagen gel, or a liquid collagen solution. When used in solution, collagen concentration may vary between about 0.1 mg/mL and about 100 mg/mL, about 1 mg/mL and about 10 mg/mL, about 10 mg/mL and about 20 mg/mL, about 20 mg/mL and about 40 mg/mL, about 40 mg/mL and about 70 mg/mL, about 70 mg/mL and about 100 mg/mL, and preferably at about 2 mg/mL. This liquid collagen can be cast into the braided scaffold and then lyophilized to create an open-cell sponge of collagen within and around the braided filaments. The collagen can be cross-linked to enhance stability and mechanical properties through methods such as thermal crosslinking, chemical crosslinking, vapor phase crosslinking, and other known crosslinking methods

Collagen may be used in lyophilized form. The collagen may be present in the foam or sponge at a final percent by weight (% wt) of the combined device and the foam or sponge of about 0.05% wt and about 99% wt, about 1% wt and about 90% wt, about 1% wt and about 80% wt, about 1% wt and about 70% wt, about 1% wt and about 60% wt, about 1% wt and about 50% wt, about 1% wt and about 40% wt, about 1% wt and about 30% wt, about 1% wt and about 20% wt, about 1% wt and about 15% wt, about 1% wt and about 10% wt, about 1% wt and about 7% wt, or about 5% wt.

### ii. Anti-microbial agents

Suitable anti-microbial agents include agents against viral, bacterial or fungal infection, such as, neomycin, streptomycin, chloramphenicol, cephalosporin, ampicillin, penicillin, tetracycline, and ciprofloxacin griseofulvin, ketoconazole, itraconizole, amphotericin B, nystatin, and candicidin, antihistamines/antipruritics (e.g., hydroxyzine, diphenhydramine, chlorpheniramine, brompheniramine maleate, cyproheptadine hydrochloride, terfenadine, clemastine fumarate, triprolidine, carbinoxamine, diphenylpyraline, phenindamine, azatadine, tripelennamine, dexchlorpheniramine maleate, and methdilazine); antibacterial agents (e.g., amikacin sulfate, aztreonam, chloramphenicol, chloramphenicol palmitate, ciprofloxacin, clindamycin, clindamycin palmitate, clindamycin phosphate, metronidazole, metronidazole hydrochloride, gentamicin sulfate, lincomycin hydrochloride, tobramycin sulfate, vancomycin hydrochloride, polymyxin B sulfate, colistimethate sodium, and colistin sulfate); antiviral agents (e.g., interferon alpha, beta or gamma, zidovudine, amantadine hydrochloride, ribavirin, and acyclovir); antimicrobials (e.g., cephalosporins such as cefazolin sodium, cephradine, cefaclor, cephapirin sodium, ceftizoxime sodium, cefoperazone sodium, cefotetan disodium, cefuroxime e azotil, cefotaxime sodium, cefadroxil monohydrate, cephalexin, cephalothin sodium, cephalexin hydrochloride monohydrate, cefamandole nafate, cefoxitin sodium, cefonicid sodium, ceforanide, ceftriaxone sodium, ceftazidime, cefadroxil, cephradine, and cefuroxime sodium; penicillins such as ampicillin, amoxicillin, penicillin G benzathine, cyclacillin, ampicillin sodium, penicillin G potassium, penicillin V potassium, piperacillin sodium, oxacillin sodium, bacampicillin hydrochloride, cloxacillin sodium, ticarcillin disodium, azlocillin sodium, carbenicillin indanyl sodium, penicillin G procaine, methicillin sodium, and nafcillin sodium; erythromycins such as erythromycin ethylsuccinate, erythromycin, erythromycin estolate, erythromycin lactobionate, erythromycin stearate, and erythromycin ethylsuccinate; and tetracyclines such as tetracycline hydrochloride, doxycycline hyclate, and minocycline hydrochloride, azithromycin, clarithromycin); anti-infectives (e.g., GM-CSF); steroidal compounds, hormones and hormone analogues (e.g., incretins and incretin mimetics such as GLP-1 and exenatide, androgens such as danazol, testosterone cypionate, fluoxymesterone, ethyltestosterone, testosterone enathate, methyltestosterone, fluoxymesterone, and testosterone cypionate; estrogens such as estradiol, estropipate, and conjugated estrogens; progestins such as methoxyprogesterone acetate, and norethindrone acetate; corticosteroids such as triamcinolone, betamethasone, betamethasone sodium phosphate, dexamethasone, dexamethasone sodium phosphate, dexamethasone acetate, prednisone, methylprednisolone acetate suspension, triamcinolone acetonide, methylprednisolone, prednisolone sodium phosphate, methylprednisolone sodium succinate, hydrocortisone sodium succinate, triamcinolone hexacetonide, hydrocortisone, hydrocortisone cypionate, prednisolone, fludrocortisone acetate, paramethasone acetate, prednisolone tebutate, prednisolone acetate, prednisolone sodium phosphate, and hydrocortisone sodium succinate; or thyroid hormones such as levothyroxine sodium), or a combination of two or more of these agents.

In some embodiments the foam or sponge is a combination of the synthetic polymer foam or sponge with the biological molecule.

The porous foam or sponge may be of any suitable dimension to enclose at least a part of the device.

Methods of making foams and sponges are known in the art. Exemplary methods include lyophillization, salt leaching, using super critical $CO_2$ or liquid CO2 as a blowing agent, or a chemical reaction or polymerization to form an interconnected pore structure with a pore size between about 5 $\mu$m and about 500 $\mu$m.

### c. Crosslinking

The foam or sponge may be added to the device and cross-linked. In other embodiments, the materials forming the foam or sponge may be added to the device and cross-linked.

For example, the materials forming the foam or sponge in lyophilized form, in gel form, or solubilized in any suitable liquid, such as in an aqueous buffer, may be added to the device and cross-linked. When used in a solution, the concentration for the materials may vary between about 1 ng/mL and about 1000 ng/mL, about 1 $\mu$g/mL and about 1000 $\mu$g/mL, about 1 mg/mL and about 100 mg/mL, about 10 mg/mL and about 20 mg/mL, about 20 mg/mL and about 40 mg/mL, about 40 mg/mL and about 70 mg/mL, about 70 mg/mL and about 100 mg/mL, and preferably at about 2 mg/ml, 5 mg/ml, 35 mg/mL, or 50 mg/ml. The solution may be added to the device, crosslinked, and then the device with the foam or sponge washed and dried to form a device with a three-dimensional braided scaffold at least partially embedded in a porous foam or sponge.

Any one of the foam materials, or a combination of foam materials, may be used to form the lyophilized form. The lyophilized materials may be added to the device and cross-linked. The lyophilized materials may be present in the foam or sponge at a final concentration of between about 0.05% wt and about 99% wt, about 1% wt and about 90% wt, about 1% wt and about 80% wt, about 1% wt and about 70% wt, about 1% wt and about 60% wt, about 1% wt and about 50% wt, about 1% wt and about 40% wt, about 1% wt and about 30% wt, about 1% wt and about 20% wt, about 1% wt and about 15% wt, about 1% wt and about 10% wt, about 1% wt and about 7% wt, or about 5% wt.

The foam or sponge material may be cross-linked, or be cross-linked to the fibers of the device. Exemplary materials that may be cross-linked include polyethylene glycol (PEG), poly(glycerol sebacate) (PGS), or collagen.

Methods of crosslinking of biological molecules are known in the art, and include the use of crosslinking agents such as glutaraldehyde, paraformaldehyde, and formaldehyde.

### d. Sterilization

The device alone or in combination with the foam or sponge may be sterilized by any suitable means, such as ethylene oxide sterilization, nitrogen dioxide sterilization, glutaraldehyde and formaldehyde sterilization, hydrogen peroxide sterilization, peracetic acid sterilization, gamma irradiation, and electron beam sterilization.

### e. Hydration

The foam or sponge may be hydrated with any suitable medium prior to device implantation. Exemplary hydrating media for hydrating the foam or sponge are known in the art and include buffers(such as phosphate buffered saline (PBS), phosphate buffer (PB), Hank's balanced salt solution (HBSS), cell culture media, cell storing media, serum and solutions thereof, blood, cells in culture media, bone marrow aspirates, and other similar biological compositions. The hydration of the foam or sponge and the device typically occurs before implantation of the device. In some embodiments, the hydration of the foam or sponge and the device occurs after implantation. The hydration may be initiated about 10 min, about 15 min, about 20 min, about 30 min, about 40 min, about 50 min, about 1 hour, 1.5 hours, about 2 hours, about 3 hours prior to device implantation. Alternatively, the device and the foam or sponge are stored hydrated in a sterile hydrating medium.

Typically, the foam or sponge is resorbed in between one week and three months, such as between one week and six weeks, following implantation.

### III. Methods of Making the Device

### A. Methods of Making Polymeric Filaments

Methods of forming polymeric filaments are known in the art, and include molding, such as injection molding, extrusion, spinning, including melt spinning, dry spinning, wet spinning, and electospinning, or casting (Stevens, Polymer Chemistry: an introduction, 3rd ed., 1999, Oxford University Press, New York, New York, 10016). The polymeric filaments are then twisted/plied to form multifilament fibers.

### B. Methods of Making Three-Dimensional Braded Scaffolds

The device is prepared using standard equipment and techniques for three-dimensional braiding. The equipment and techniques can be modified to create scaffolds with multiple regions (two ends for attachment and at least one, two, three or more regions).

The two major methods for braiding the three-dimensional braided scaffolds are "Cartesian" (or in its modified version cylindrical) braiding, also called "row and column" ("ring and column", respectively) and a "rotary" (also known as "homgear") braiding. The geometric parameters which determine the shape and fiber architecture of three-dimensional braids includes braiding angle distribution, yarn volume fraction, number of carriers, number of centers, and braiding yarn width.

A track plate is kept at the bottom of the braiding machine. Packages, which supply axial yarns, are kept beneath the track plate. Bobbins are mounted on the carrier, which moves over the track plate. Braiding yarns are fed from these bobbins.

In circular braiding, the bobbins (with opposite directions of rotation) move in two concentric orbits. The two orbits interfere to form dephased sinusoidal oscillations that determine the thread's pattern and crossing point. At this crossing point, the bobbins change their path to produce the upper and inner side of the braid. Generally, the circular braiding process produces braids with rotational symmetry. The over-braiding process follows the same principle as the circular braiding process, but the only modification is that the crossing point is located at the center.

In the four-step braiding process, the bobbins move on the X and Y axes, which are mutually perpendicular to each other. In each step, the bobbins move to the neighboring crossing point in two ways and stop for a specific interval of time. Basic arrangement of the braiding field is obtained after a minimum of four steps.

In the two-step braiding process, the bobbins move continuously without stopping. They move on the track plate through the complete structure and around the standing ends, such that the movements of bobbins are faster when compared to the four-step braiding process. The bobbins can move only in two directions, so the process is called the two-step braiding process.

The 3D rotary braiding process consists of base plates with horn gears and mobile bobbins arranged upon them.

Switches are used to control the position of the threads and horn gears.

### C. Incorporating Braid Inserts into the Device

During braiding, the braid insert is incorporated into the three-dimensional braided scaffold via center filament(s) passing through the braid insert's axial through hole. One insert at a time is brought down via the center filaments to a desired location and is tied with the braiding filaments via the horizontal through holes. This secures the braid insert at the desired location.

### IV. Kits

The devices are typically provided in a sterile kit, such as a foil or TYVEX® package. In a preferred embodiment, the device will include sutures or whip stitching in the graft, to facilitate placement. A kit containing a device at least partially embedded in a foam or a sponge is also provided. A kit containing a device at least partially embedded in a foam or a sponge may include a suitable sterile hydrating medium and a container for holding and hydrating the device with the foam or the sponge. Any suitable container may be provided, such as a tray, a jar, a bowl, a tube, and so on.

The kit will typically include necessary hardware and equipment for implantation and fixation of the devices to tissues, including commercially available fixation hardware, such as fixation screws, suspensory fixation tools, suture and whipstitching, ENDOBUTTON®, or other suspensory fixation equipment.

In addition, the kits may contain equipment for extraction and isolation of patient's own tissue, fluids, fractions, or cells. Such equipment includes sterile syringes of suitable size and sterile needles of suitable size for extraction of patient-derived bone marrow cells, epithelial stem cells, muscle mesenchymal cells. The kit can also contain culture flasks sized to accommodate the devices of the kits, as well growth media with growth factors to support the *in vitro* growth and proliferation of the extracted cells. The kits may also contain solutions and equipment for pre-treatment and sterilization of scaffolds prior to cell seeding and culturing. Kits may also include equipment for seeding and/or incubating the scaffold with fluids containing patient derived substances.

### V. Methods of Use

The device is used for regeneration or reconstruction of musculoskeletal tissue injuries, including articular tissue injuries. The polymeric devices are implanted a site in need of reconstruction.

Devices with biomechanical properties and degradation rates that mimic the biomechanical properties and reconstruction rates of the injured tissues can be implanted into a subject in need thereof. The devices permit an early return to normal function post-operatively. The implanted device bears applied loads and tissue in-growth commences. The mechanical properties of the biodegradable material of the implant slowly decay following implantation, to permit a gradual transfer of load to the ingrown fibrous tissue. Additional in-growth continues into the space provided by the biodegradable material of the implant as it is absorbed. This process continues until the biodegradable material is completely absorbed and only the newly formed tissue remains. In a preferred embodiment, the degradation of the biodegradable material is complete after about 9-12 months post-implantation.

After implantation at a site in the tissue in need of reconstruction, the device is affixed to the tissue in need of reconstruction, or to nearby tissues. Affixing the devices can be achieved using techniques within the purview of those skilled in the art using, for example, sutures, staples, pins or screws, with or without the use of appropriate anchors, pledgets, and other commercially available fixation hardware, such as fixation screws, suspensory fixation tools, suture & whipstitching, ENDOBUTTON®, or other suspensory fixation equipment.

In other embodiments, the devices can be used to culture patient-derived bone marrow cells, epithelial stem cells, mesenchymal cells, or other pluripotent or multipotent cells, *in vitro,* to form auto/allogeneic biologic constructs. After culturing, the biologic construct containing some or no polymer can be treated to improve storage stability prior to implantation, and integration following implantation. Suitable treatments can 'de-cellularize' the construct to remove immunogenic factors, and/or sterilize the construct to enhance the construct's shelf-life and/or ease its integration following implantation. Desirable treatment processes would typically remove or inactivate a wide range of bacterial, viral and fungal pathogens, be non-toxic to the biological construct and the host, retain or improve desirable characteristics of the construct, such as its biomechanical strength or growth-inducing properties, and be effective across a wide range of scaffolds designed for a wide variety of tissue types. Suitable methods are known in the art and are discussed in U.S. Patent Application Publication No. US 2013/0302435 and U.S. Patent No. 8,563,232.

### A. Musculoskeletal System Reconstruction

The device is used in the regeneration, reconstruction or augmentation of bones of the skeleton, muscles, cartilage, tendons, ligaments, joints, and other connective tissues. The devices are particularly useful at reconstruction and regeneration of articular tissues, such as the ligaments and tendons.

Articular injuries include both intra-articular and extra-articular injuries. Intra-articular injuries involve, for example, injuries to meniscus, ligament and cartilage. Extra-articular injuries include, but are not limited to, injuries to the ligament, tendon or muscle. These include injuries to the Anterior cruciate ligament (ACL), Lateral collateral ligament (LCL), Posterior cruciate ligament (PCL), Medial collateral ligament (MCL), Volar radiocarpal ligament, Dorsal radiocarpal ligament, Ulnar collateral ligament, Radial collateral ligament, meniscus, labrum, for example glenoid labrum and acetabular labrum, cartilage, and other tissues. The injury being treated may be a torn or ruptured ligament.

A ligament is a short band of tough fibrous connective tissue composed of collagen fibers. Ligaments connect bones to other bones to form a joint. A torn ligament is one where the ligament remains connected but has been damaged causing a tear in the ligament. The tear may be of any length or shape. A ruptured ligament is one where the ligament has been completely severed providing two separate ends of the ligament. A ruptured ligament may provide two ligament ends of similar or different lengths. The rupture may be such that a ligament stump is formed at one end.

A tendon is a tough, flexible band of fibrous connective tissue that connects muscles to bones. Tendons consist of soft and fibrous connective tissue that is composed of densely packed collagen fiber bundles aligned parallel to the longitudinal tendon axis and surrounded by a tendon sheath, also consisting of extracellular matrix (ECM) components. Tendon tissue has a crimped, waveform appearance under microscopic observation. Collagen type I constitutes approximately 60% of the dry mass of the tendon.

As the tendon inserts into the proximal bone, there is a gradual transitional zone with four distinct properties at the direct tendon-to-bone insertion: tendon, nonmineralized fibrocartilage, mineralized fibrocartilage and bone. Replicating this normal transitional zone in the devices will prevent failure of the implanted devices from the stress concentrations that weaken the healed tendon-to-bone insertion site. The stress concentrations are mainly attributed to the mechanical mismatch between the tendon, a soft tissue with a modulus of 200 MPa, and bone, with a modulus of 20 GPa, one of the biggest mechanical mismatches in nature.

The braiding design, the fiber architecture, and the polymeric composition of the filaments used herein can form three-dimensional braided scaffolds with complex braided structure and multiple regions, so that the regions differ in their mechanical properties and degradation rates. For example, a single scaffold may be braided from yarns that are rapidly and gradually degrading, while the end regions for attachment to bones may incorporate yarns that are very slowly degrading. The end regions may also be braided at tighter braiding angle than the middle region, and/or incorporate additional components, such as tricalcium phosphate, hydroxyapatite, fluorapatite, aluminum oxide, zirconium oxide, or other porous mineral/polymer composites, to increase the elastic modulus of the end regions of the scaffold and replicate the normal transition zone of fibrocartilage to bone.

### 1. Devices for Use in ACL Reconstruction

The devices can be useful for ligament and tendon reconstruction. An exemplary method is ACL reconstruction using a device described in Figures 3-5B.

Implantation of the device is performed using standard arthroscopic technique with two or three portals. A guide is drilled through the tibia with overdrill using a reamer on the tibia to tibial footprint area of the ACL, followed by insertion of the guide pin to the isometric point position over the femur near insertion of femoral insertion of the ACL. This is followed by an overdrill using a standard reamer on the femoral side. A Beath pin is then often used to complete drilling and the graft is then passed through the tibia and the femur. The graft is then secured using interference screws, or through the ENDOBUTTON® type technique. Visualization can be assisted using an arthrotomy. Preferably the graft is placed without an arthrotomy for a completely arthroscopically based technique.

The ends **32a, 32b** are placed in bone tunnels and secured with interference screws, rivets, sutures **36** or other techniques, as shown in Figures 5A and 5B. The device is intended to be implanted using the same techniques currently used by surgeons to implant patella tendon grafts.

The following non-limiting examples are provided to further illustrate the present invention.

Examples

### Example 1. Complex three-dimensional braided scaffolds formed with various braiding designs.

Materials and methods

A four-track three-dimensional braiding technique was used to generate the tissue reconstruction devices. The yarns used in the carriers are monofilament fibers, multifilament yarns, composite multifilament yarns, composite yarns, or combinations thereof. See Figures 1A-1G.

Sixty (60) such filaments were twisted together to form one multifilament fiber, and 24 such multifilament fibers were twisted together to form one multifilament yarn (24-end x60 multifilament). Composite yarns used were multifilament fibers and monofilament fibers twisted together. Here 40 PLLA filaments each with 15 micron in diameter and one monofilament fiber were twisted around each other to form the composite yarns (mono/multi composite).

Results

The designs for yarn arrangement in the three-dimensional braider are presented in Figures 2A-2Q. The designs include monofilament-only designs for three-dimensional braiding with multifilament-only designs for braiding with multifilament yarns only (Figures 2A-2F), as well as designs that include any combination of multifilament yarns and composite yarns (Figures 2G-2Q), in the presence or absence (Figures 2A, 2G, 2I, 2M-MO) of centers.
Embodiments according to the claims comprise centers which are pulled into the three dimensional braided scaffold between carrier carrying bobbins with yarn bundles where the centers are not braided, the centers containing fibers and/or yarns of any structure and composition.

**Example 2. Mechanical properties of complex three-dimensional braids.**

Materials and Methods

Two different complex braids, designated 'D3' and 'D5' were constructed.
D3 was made with 100% twisted P4HB multifilament yarns on all braider bobbins, with twisted PGA multifilament fibers and P4HB monofilament fiber bundles brought into the braid via 17 different center locations.
D5 was made with 56% of braider bobbins containing twisted P4HB multifilament and monofilament fiber bundles, 44% of braider bobbins containing only P4HB multifilament fiber, and PGA multifilament fiber bundles brought into braid via 17 center locations.
These devices were then mechanically tested at time zero, and following incubation in PBS over 1 year.

Results

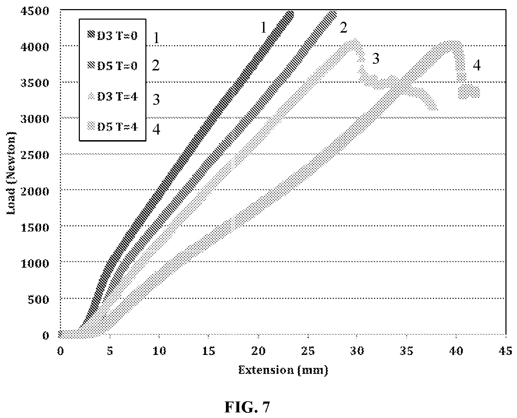

D3 and D5 have similar chemical composition but differ in architecture, creating significant differences in mechanical properties.
After 4 weeks, devices containing PGA have reduced stiffness, indicating a 2 stage resorption profile as the PGA is degraded rapidly and before P4HB (Figure 7). A control group of devices containing 100% P4HB did not lose any stiffness over the first 4 weeks.
This embodiment is designed for use in conditions such as ALC reconstruction. where the graft is first quite stiff, helping stabilize the patient knee, and then reducing in stiffness as patient tissue is developed.

**Claims**

1.    A device for replacement or regeneration of tendons and ligaments comprising

    a complex three-dimensional braided scaffold comprising
    polymeric multifilament yarn bundles, composite multifilament yarn bundles, composite yarn bundles, or combinations thereof,
    braided into a three-dimensional braided scaffold to form at least one end region for mechanical fixation of the device at a site of implantation and at least one tissue region for tendon or ligament tissue regeneration, and centers which are pulled into the three-dimensional braided scaffold between carriers carrying bobbins with yarn bundles where the centers are not braided, the centers containing fibers and/or yarns of any structure and composition,
    wherein the device has an initial stiffness of between about 100 and 750 N/mm, an ultimate strength of between about 1200 N and 6500 N, and greater than 10% elongation at yield at implantation, and
    provides tissue function at the site of implantation as tissue integrates into the device,
    wherein the device comprises multifilament fibers, monofilament fibers, composite multifilament yarns, composite yarns, braided or twisted fibers, or any combination thereof, sewn, stitched, tied, or welded to the outside of the device and between the end region and the tissue region.

2.    The device of claim 1, wherein

the polymeric multifilament yarn bundles are twisted or plied bioresorbable multifilament fibers with similar composition, diameter and denier,

the composite multifilament yarn bundles are twisted or plied bioresorbable multifilament fibers that differ from each other in composition, diameter and/or denier, and

the composite yarn bundles are twisted or plied bioresorbable multifilament and monofilament fibers.

3. The device of claim 1 or 2, comprising between 1 and 500 multifilament fibers, monofilament fibers, multifilament yarns, composite multifilament yarns, composite yarns, braided or twisted fibers, or any combination thereof, incorporated unbraided through the scaffold.

4. The device of claim 1 or 2, further comprising multifilament fibers, monofilament fibers, composite multifilament yarns, composite yarns, braided or twisted fibers, or any combination thereof, sewn, stitched, tied, or welded throughout one or more regions of the device.

5. The device of any one of claims 1-4, wherein the device has a total length of between about 5 and 15 cm, and a radius of the cross-sectional area of between about 1 and 8 mm, and either

(a) the device loses about 90% of its initial ultimate strength within about 3 to 12 months following implantation or
(b) the device loses at least 50% of its mass within about 3 to 18 months following implantation.

6. The device of any one of claims 1-5, wherein the end region differs from the tissue region in one or more of fiber structure, fiber twisting, fiber plying, yarn structure, yarn twisting, yarn plying, polymer composition, surface chemistry, braiding angle, porosity, void space volume, packing density, size, shape, tension, mechanical strength, and degradation rate.

7. The device of any one of claims 1-6, wherein the end region has greater than 20% void space with no constriction smaller than about 3 microns, and
the tissue region has greater than 40% void space with no constriction smaller than about 3 microns.

8. The device of any one of claims 1-7, wherein the end region is attachable to bone and allows for ingrowth of bony tissue and connective tissue, and wherein the tissue region allows ingrowth of soft tissue and connective tissue.

9. The device of any one of claims 1-8 having, prior to implantation, a peak load strength between about 1200 N and 6500 N, and elastic modulus ranging from between 0.1 MPa and 10 GPa when measured at room temperature and humidity.

10. The device of any one of claims 1-9 wherein the number of filaments per bundle is between 10 and 2500 and the number of bundles per braid is between 12 and 64.

11. The device of any one of claims 1-10, wherein the multifilament fibers are formed of filaments with a diameter between 1 micron and 100 microns, and the fiber denier between 1 and 1000,
optionally wherein at least 50% of the multifilament fibers have filament diameters of between 1 and 40 microns.

12. The device of any one of claims 1-11 wherein the polymer fibers comprise polymers selected from the group consisting of poly(hydroxy esters), poly(alpha-hydroxy acids), poly(beta-hydroxy acids), poly(gamma-hydroxy acids), poly(delta-hydroxy acids), poly(epsilon-hydroxy acids), polylactides, poly(lactic acids), poly(glycolic acids), polycaprolactones, poly(hydroxybutyrates), poly(3-hydroxybutyrate), poly(4-hydroxybutyrate), poly(trimethylene carbonate) (PTMC), polydioxanone (PDO), poly(butylene succinate) (PBS), polyorthoesters, polyanhydrides, polyphosphazenes, polyhydroxyalkanoates, biodegradable polyurethanes, polyanhydride-co-imides, polypropylene fumarates, polysaccharides, collagen, silk, chitosan, celluloses, copolymers or blends thereof.

13. The device of any one of claims 1-12, wherein the device is seeded, for regeneration or reconstruction of an ACL ligament, prior to implantation with patient-derived tissue, blood, or bone marrow derived product such as platelet rich plasma, platelets, mononuclear cells, progenitor cells, inflammatory cells, primary cells, or purified cell populations, fluids, or proteins, and/or cells selected from any of mesenchymal cells, cells generating mesenchymal cells, fibroblasts, tenocytes, pluripotent stem cells, and multipotent stem cells.

14. A method of making the device of any of claims 1-13 comprising

braiding a three-dimensional scaffold using polymeric multifilament yarns, polymeric composite multifilament yarns, polymeric composite yarns, or any combination thereof, mounted on any combination of between 3 and 128 carriers carrying bobbins with yarn bundles using a braider,

pulling in centers into the three-dimensional scaffold between the carriers where the centers are not braided, the centers containing fibers and/or yarns of any structure and composition,

the polymeric yarns braided into a three-dimensional braided scaffold to form at least one end section for attachment of the device at a site of implantation and at least one tissue section for regeneration of soft tissue, wherein the device performs the function of the tendon or ligament at the site of implantation as tissue integrates into the device, optionally wherein the device comprises between 1 and 50 multifilament fibers, monofilament fibers, multifilament yarns, composite multifilament yarns, composite yarns, braided or twisted fibers, or any combination thereof, incorporated into the scaffold through the centers of the three-dimensional scaffold.

**Patentansprüche**

1. Vorrichtung zum Ersatz oder zur Regeneration von Sehnen und Bändern, umfassend

    ein komplexes dreidimensionales Flechtgerüst, umfassend

    polymere Multifilament-Garnbündel, Multifilament-Verbundgarnbündel, Verbundgarnbündel oder Kombinationen davon, geflochten zu einem dreidimensionalen Flechtgerüst unter Ausbilden mindestens eines Endbereichs für eine mechanische Fixierung der Vorrichtung an einem Implantationsort und mindestens eines Gewebebereichs zur Regeneration von Sehnenoder Bandgewebe, und

    Zentren, die zwischen Trägern, die Spulen mit Garnbündeln tragen, in das dreidimensionale Flechtgerüst eingezogen sind, wo die Zentren nicht geflochten sind, wobei die Zentren Fasern und/oder Garne beliebiger Struktur und Zusammensetzung enthalten,

    wobei die Vorrichtung bei der Implantation eine Anfangssteifigkeit zwischen etwa 100 und 750 N/mm, eine Anfangsbruchfestigkeit zwischen etwa 1200 N und 6500 N und eine Anfangsstreckdehnung von mehr als 10 % aufweist und während des Integrierens von Gewebe in die Vorrichtung eine Gewebefunktion an dem Implantationsort bereitstellt,

    wobei die Vorrichtung Multifilamentfasern, Monofilamentfasern, Multifilament-Verbundgarne, Verbundgarne, geflochtene oder gezwirnte Fasern oder eine beliebige Kombination davon umfasst, die an die Außenseite der Vorrichtung und zwischen dem Endbereich und dem Gewebebereich genäht, geheftet, gebunden oder geschweißt sind.

2. Vorrichtung nach Anspruch 1, wobei

    die polymeren Multifilament-Garnbündel gezwirnte oder gefachte bioresorbierbare Multifilamentfasern mit ähnlicher Zusammensetzung, ähnlichem Durchmesser und ähnlichem Denier sind,

    die Multifilament-Verbundgarnbündel gezwirnte oder gefachte bioresorbierbare Multifilamentfasern sind, die sich in Zusammensetzung, Durchmesser und/oder Denier unterscheiden, und

    die Verbundgarnbündel gezwirnte oder gefachte bioresorbierbare Multifilament- und Monofilamentfasern sind.

3. Vorrichtung nach Anspruch 1 oder 2, umfassend zwischen 1 und 500 Multifilamentfasern, Monofilamentfasern, Multifilamentgarne, Multifilament-Verbundgarne, Verbundgarne, geflochtene oder gezwirnte Fasern oder eine beliebige Kombination davon, die ungeflochten durch das Gerüst hindurch eingearbeitet sind.

4. Vorrichtung nach Anspruch 1 oder 2, ferner umfassend Multifilamentfasern, Monofilamentfasern, Multifilament-Verbundgarne, Verbundgarne, geflochtene oder gezwirnte Fasern oder eine beliebige Kombination davon, die überall in einem oder mehreren Bereichen der Vorrichtung genäht, geheftet, gebunden oder geschweißt sind.

5. Vorrichtung nach einem der Ansprüche 1-4, wobei die Vorrichtung eine Gesamtlänge zwischen etwa 5 und 15 cm und einen Radius der Querschnittsfläche zwischen etwa 1 und 8 mm aufweist und entweder

    (a) das Produkt innerhalb von etwa 3 bis 12 Monaten nach Implantation etwa 90 % seiner Anfangsbruchfestigkeit verliert oder

    (b) das Produkt innerhalb von etwa 3 bis 18 Monaten nach Implantation mindestens 50 % seiner Masse verliert.

6. Vorrichtung nach einem der Ansprüche 1-5, wobei sich der Endbereich von dem Gewebebereich in einer oder mehreren von Faserstruktur, Faserzwirnung, Faserfachung, Garnstruktur, Garnzwirnung, Garnfachung, Polymerzusammensetzung, Oberflächenchemie, Flechtwinkel, Porosität, Hohlraumvolumen, Packungsdichte, Größe, Form, Spannung, mechanischer Festigkeit und Abbaugeschwindigkeit unterscheidet.

7. Vorrichtung nach einem der Ansprüche 1-6, wobei der Endbereich mehr als 20 % Hohlraum ohne Verengung kleiner als etwa 3 Mikrometer aufweist und
die Geweberegion mehr als 40 % Hohlraum ohne Verengung kleiner als etwa 3 Mikrometer aufweist.

8. Vorrichtung nach einem der Ansprüche 1-7, wobei der Endbereich an Knochen anbringbar ist und ein Einwachsen von Knochengewebe und Bindegewebe ermöglicht und wobei der Gewebebereich Einwachsen von Weichgewebe und Bindegewebe ermöglicht.

9. Vorrichtung nach einem der Ansprüche 1-8, aufweisend eine Spitzenbelastbarkeit zwischen etwa 1200 N und 6500 N und einen Elastizitätsmodul im Bereich von 0,1 MPa bis 10 GPa, gemessen bei Raumtemperatur und Feuchtigkeit, vor Implantation.

10. Vorrichtung nach einem der Ansprüche 1-9, wobei die Anzahl von Filamenten pro Bündel zwischen 10 und 2500 und die Anzahl von Bündeln pro Geflecht zwischen 12 und 64 beträgt.

11. Vorrichtung nach einem der Ansprüche 1-10, wobei die Multifilamentfasern aus Filamenten mit einem Durchmesser zwischen 1 Mikrometern und 100 Mikrometern und einem Faserdenier zwischen 1 und 1000 ausgebildet sind, wobei optional mindestens 50 % der Multifilamentfasern einen Filamentdurchmesser zwischen 1 und 40 Mikrometern aufweisen.

12. Vorrichtung nach einem der Ansprüche 1-11, wobei die Polymerfasern Polymere umfassen, ausgewählt aus der Gruppe, bestehend aus Poly(hydroxyestern), Poly(alpha-hydroxysäuren), Poly(beta-hydroxysäuren), Poly(gamma-hydroxysäuren), Poly(delta-hydroxysäuren), Poly(epsilon-hydroxysäuren), Polylactiden, Poly(milchsäuren), Poly(glykolsäuren), Polycaprolactonen, Poly(hydroxybutyraten), Poly(3-hydroxybutyrat), Poly(4-hydroxybutyrat), Poly(trimethylencarbonat) (PTMC), Polydioxanon (PDO), Poly(butylensuccinat) (PBS), Polyorthoestern, Polyanhydriden, Polyphosphazenen, Polyhydroxyalkanoaten, biologisch abbaubaren Polyurethanen, Polyanhydrid-Co-Imiden, Polypropylenfumaraten, Polysacchariden, Kollagen, Seide, Chitosan, Cellulosen, Copolymere oder Mischungen davon.

13. Vorrichtung nach einem der Ansprüche 1-12, wobei die Vorrichtung zur Regeneration oder Rekonstruktion eines Kreuzbands vor Implantation mit einem vom Patienten stammenden aus Gewebe, Blut oder Knochenmark abgeleiteten Produkt, wie thrombozytenreichem Plasma, Thrombozyten, mononukleären Zellen, Vorläuferzellen, Entzündungszellen, Primärzellen oder gereinigten Zellpopulationen, Fluiden oder Proteinen und/oder Zellen, die aus mesenchymalen Zellen, Zellen, die mesenchymale Zellen erzeugen, Fibroblasten, Tenozyten, pluripotenten Stammzellen und multipotenten Stammzellen ausgewählt sind, beimpft ist.

14. Verfahren zur Herstellung der Vorrichtung nach einem der Ansprüche 1-13, umfassend

Flechten eines dreidimensionalen Gerüsts unter Verwendung von polymeren Multifilamentgarnen, polymeren Multifilament-Verbundgarnen, polymeren Verbundgarnen oder einer beliebigen Kombination davon, die auf einer beliebigen Kombination von 3 bis 128 Trägern montiert sind, die Spulen mit Garnbündeln tragen, unter Verwendung einer Flechtmaschine,
Einziehen von Zentren in das dreidimensionale Gerüst zwischen den Trägern, wo die Zentren nicht geflochten sind, wobei die Zentren Fasern und/oder Garne beliebiger Struktur und Zusammensetzung enthalten,
wobei die Polymergarne unter Ausbilden mindestens eines Endabschnitts zur Anbringung der Vorrichtung an einem Implantationsort und mindestens eines Gewebeabschnitts zur Regeneration von Weichgewebe zu einem dreidimensionalen Flechtgerüst geflochten werden,
wobei die Vorrichtung während des Integrierens von Gewebe in die Vorrichtung die Funktion der Sehne oder des Bandes an dem Implantationsort durchführt, wobei die Vorrichtung optional zwischen 1 und 50 Multifilamentfasern, Monofilamentfasern, Multifilamentgarne, Multifilament-Verbundgarne, Verbundgarne, geflochtene oder gezwirnte Fasern oder eine beliebige Kombination davon umfasst, die durch die Zentren des dreidimensionalen Gerüsts in das Gerüst eingearbeitet sind.

**Revendications**

1. Dispositif de remplacement ou de régénération de tendons et de ligaments comprenant

   un échafaudage tressé tridimensionnel complexe comprenant
   des faisceaux de fils multifilaments polymères, des faisceaux de fils multifilaments composites, des faisceaux de fils composites ou des combinaisons de ceux-ci,
   tressés en un échafaudage tressé tridimensionnel pour former au moins une région d'extrémité pour la fixation mécanique du dispositif sur un site d'implantation et au moins une région tissulaire pour la régénération du tissu tendineux ou ligamentaire, et
   des centres qui sont tirés dans l'échafaudage tressé tridimensionnel entre des supports portant des bobines avec des faisceaux de fils où les centres ne sont pas tressés, les centres contenant des fibres et/ou des fils de toute structure et composition,
   dans lequel le dispositif présente une rigidité initiale comprise entre environ 100 et 750 N/mm, une résistance ultime comprise entre environ 1 200 N et 6 500 N, et un allongement à la limite d'élasticité supérieur à 10 % lors de l'implantation, et
   assure la fonction tissulaire sur le site d'implantation lorsque le tissu s'intègre dans le dispositif,
   dans lequel le dispositif comprend des fibres multifilaments, des fibres monofilaments, des fils multifilaments composites, des fils composites, des fibres tressées ou torsadées, ou toute combinaison de ceux-ci, cousus, piqués, noués ou soudés à l'extérieur du dispositif et entre la région d'extrémité et la région tissulaire.

2. Dispositif selon la revendication 1, dans lequel

   les faisceaux de fils multifilaments polymères sont des fibres multifilaments biorésorbables torsadées ou pliées ayant une composition, un diamètre et un denier similaires,
   les faisceaux de fils multifilaments composites sont des fibres multifilaments biorésorbables torsadées ou pliées qui diffèrent les unes des autres par leur composition, leur diamètre et/ou leur denier, et
   les faisceaux de fils composites sont des fibres multifilaments et monofilaments biorésorbables torsadées ou pliées.

3. Dispositif selon la revendication 1 ou 2, comprenant entre 1 et 500 fibres multifilaments, fibres monofilaments, fils multifilaments, fils multifilaments composites, fils composites, fibres tressées ou torsadées, ou toute combinaison de ceux-ci, incorporés non tressés à travers l'échafaudage.

4. Dispositif selon la revendication 1 ou 2, comprenant également des fibres multifilaments, des fibres monofilaments, des fils multifilaments composites, des fils composites, des fibres tressées ou torsadées, ou toute combinaison de ceux-ci, cousus, piqués, noués ou soudés dans une ou plusieurs régions du dispositif.

5. Dispositif selon l'une quelconque des revendications 1 à 4, dans lequel le dispositif a une longueur totale comprise entre environ 5 et 15 cm, et un rayon de section transversale compris entre environ 1 et 8 mm, et

   (a) le dispositif perd environ 90 % de sa résistance ultime initiale dans les 3 à 12 mois suivant l'implantation ou
   (b) le dispositif perd au moins 50 % de sa masse dans les 3 à 18 mois suivant l'implantation.

6. Dispositif selon l'une quelconque des revendications 1 à 5, dans lequel la région d'extrémité diffère de la région de tissu d'un ou plusieurs éléments parmi la structure de fibre, la torsion de fibre, le pliage de fibre, la structure de fil, la torsion de fil, le pliage de fil, la composition de polymère, la chimie de surface, l'angle de tressage, la porosité, le volume d'espace vide, la densité de tassement, la taille, la forme, la tension, la résistance mécanique et le taux de dégradation.

7. Dispositif selon l'une quelconque des revendications 1 à 6, dans lequel la région d'extrémité présente plus de 20 % d'espace vide sans rétrécissement inférieur à environ 3 microns, et
   la région de tissue présente plus de 40 % d'espace vide sans constriction inférieure à environ 3 microns.

8. Dispositif selon l'une quelconque des revendications 1 à 7, dans lequel la région d'extrémité peut être fixée à un os et permet la croissance de tissu osseux et de tissu conjonctif, et dans lequel la région tissulaire permet la croissance de tissu mou et de tissu conjonctif.

9. Dispositif selon l'une quelconque des revendications 1 à 8, ayant, avant l'implantation, une résistance à la charge maximale comprise entre environ 1 200 N et 6 500 N, et un module d'élasticité compris entre 0,1 MPa et 10 GPa lorsqu'ils sont mesurés à température et humidité ambiantes.

10. Dispositif selon l'une quelconque des revendications 1 à 9, dans lequel le nombre de filaments par faisceau est compris entre 10 et 2 500 et le nombre de faisceaux par tresse est compris entre 12 et 64.

11. Dispositif selon l'une quelconque des revendications 1 à 10, dans lequel les fibres multifilaments sont constituées de filaments d'un diamètre compris entre 1 micron et 100 microns, et d'un denier de fibre compris entre 1 et 1000, éventuellement dans lequel au moins 50 % des fibres multifilaments ont des diamètres de filament compris entre 1 et 40 microns.

12. Dispositif selon l'une quelconque des revendications 1 à 11, dans lequel les fibres polymères comprennent des polymères choisis dans le groupe constitué par les poly(hydroxyesters), les poly(alpha-hydroxyacides), les poly(bêta-hydroxyacides), les poly(gamma-hydroxyacides), les poly(delta-hydroxyacides), les poly(epsilon-hydroxyacides), les polylactides, les poly(acides lactiques), les poly(acides glycoliques), les polycaprolactones, les poly(hydroxybutyrates), le poly(3-hydroxybutyrate), le poly(4-hydroxybutyrate), le poly(triméthylène carbonate) (PTMC), la polydioxanone (PDO), le poly(succinate de butylène) (PBS), les polyorthoesters, les polyanhydrides, les polyphosphazènes, les polyhydroxyalcanoates, les polyuréthanes biodégradables, les polyanhydrides-co-imides, les fumarates de polypropylène, les polysaccharides, le collagène, la soie, le chitosane, les celluloses, les copolymères ou les mélanges de ceux-ci.

13. Dispositif selon l'une quelconque des revendications 1 à 12, dans lequel le dispositif est ensemencé, pour la régénération ou la reconstruction d'un ligament LCA, avant l'implantation avec un tissu dérivé du patient, du sang ou d'un produit dérivé de la moelle osseuse tel que du plasma riche en plaquettes, des plaquettes, des cellules mononucléaires, des cellules progénitrices, des cellules inflammatoires, des cellules primaires ou des populations cellulaires purifiées, des fluides ou des protéines, et/ou des cellules choisies parmi les cellules mésenchymateuses, les cellules génératrices de cellules mésenchymateuses, les fibroblastes, les ténocytes, les cellules souches pluripotentes et les cellules souches multipotentes.

14. Procédé de fabrication du dispositif selon l'une quelconque des revendications 1 à 13, comprenant

le tressage d'un échafaudage tridimensionnel à l'aide de fils multifilaments polymères, de fils multifilaments composites polymères, de fils composites polymères ou de toute combinaison de ceux-ci, montés sur toute combinaison comprise entre 3 et 128 supports portant des bobines avec des faisceaux de fils à l'aide d'une tresseuse,
la traction des centres dans l'échafaudage tridimensionnel entre les supports où les centres ne sont pas tressés, les centres contenant des fibres et/ou des fils de toute structure et composition,
les fils polymères étant tressés dans un échafaudage tressé tridimensionnel pour former au moins une section d'extrémité pour la fixation du dispositif à un site d'implantation et au moins une section de tissu pour la régénération des tissus mous, dans lequel le dispositif remplit la fonction du tendon ou du ligament au niveau du site d'implantation à mesure que le tissu s'intègre dans le dispositif, éventuellement dans lequel le dispositif comprend entre 1 et 50 fibres multifilaments, fibres monofilaments, fils multifilaments, fils multifilaments composites, fils composites, fibres tressées ou torsadées, ou toute combinaison de ceux-ci, incorporés dans l'échafaudage à travers les centres de l'échafaudage tridimensionnel.

10 →

**FIG. 1A**

12 →

**FIG. 1B**

16 →

**FIG. 1D**

20 →

**FIG. 1F**

14 →

**FIG. 1C**

18 →

**FIG. 1E**

22 →

**FIG. 1G**

Track 1       Track 2       Track 3

Empty Centers

Track 4

* 36 carriers:
    24-end x 60-multifilament
* No centers

**FIG. 2A**

Track 1       Track 2       Track 3

Empty Centers
4 Mono Centers

Track 4

Design 2
* 36 carriers:
    24-end x 60-multifilament
* 4 centers:
    1, 5-0 Monofilament

**FIG. 2B**

Design 3
* 36 carriers:
    24-end x 60-multifilament
* 8 centers:
    1, 5-0 Monofilament

**FIG. 2C**

Design 4
* 36 carriers:
    24-end x 60-multifilament
* 12 centers:
    1, 5-0 Monofilament

**FIG. 2D**

Track 1    Track 2    Track 3

Empty Centers
16 Centers

Track 4

Design 5
• 36 carriers:
  24-end x 60-multifilament
• 16 centers:
  1, 5-0 Monofilament

**FIG. 2E**

Track 1    Track 2    Track 3

Empty Centers
21 Centers

Track 4

Design 6
• 36 carriers:
  24-end x 60-multifilament
• 21 centers:
  1, 5-0 Monofilament

**FIG. 2F**

Track 1          Track 2          Track 3

Empty Centers

Mono Centers                                                    Not symm
                                                               carriers – less
Multi Carriers                                                 of these
Tracks 2&4

Composite Carriers                                    Track 4
Tracks 2&4

Design 7
• 33 carriers:
    24-end x 60-multifilament
• 3 carriers:
    Mono/multi composite
• No centers

**FIG. 2G**

Track 1          Track 2          Track 3

Empty Centers

Mono Centers                                                    Not symm
                                                               carriers – less
Multi Carriers                                                 of these
Tracks 2&4

Composite Carriers                                    Track 4
Tracks 2&4

Design 8
• 33 carriers:
    24-end x 60-multifilament
× 3 carriers:
    Mono/multi composite
× 12 centers

**FIG. 2H**

Track 1          Track 2          Track 3

Empty Centers

Mono Centers

Multi Carriers
Tracks 2&4

Composite Carriers
Tracks 2&4

Symm carriers –
more of these

Track 4

Design 9
- 30 carriers:
  24-end x 60-multifilament
- 6 carriers:
  Mono/multi composite
- No centers

**FIG. 2I**

Track 1          Track 2          Track 3

Empty Centers

Mono Centers

Multi Carriers
Tracks 2&4

Composite Carriers
Tracks 2&4

Symm carriers –
more of these

Track 4

Design 10
- 30 carriers:
  24-end x 60-multifilament
- 6 carriers:
  Mono/multi composite
- 4 centers

**FIG. 2J**

Track 1          Track 2          Track 3

Empty Centers

Mono Centers

Multi Carriers
Tracks 2&4

Composite Carriers
Tracks 2&4

Symm carriers –
more of these

Track 4

Design 11
- 30 carriers:
  24-end x 60-multifilament
- 6 carriers:
  Mono/multi composite
- 8 centers

**FIG. 2K**

Track 1          Track 2          Track 3

Empty Centers

Mono Centers

Multi Carriers
Tracks 2&4

Composite Carriers
Tracks 2&4

Symm carriers –
more of these

Track 4

Design 12
- 30 carriers:
  24-end x 60-multifilament
- 6 carriers:
  Mono/multi composite
- 12 centers

**FIG. 2L**

Track 1      Track 2      Track 3

Empty Centers

Mono Centers

Multi Carriers
Tracks 2&4

Composite Carriers
Tracks 2&4

Not symm
carriers – less
of these

Track 4

Design 13
* 24 carriers:
  24-end x 60-multifilament
* 12 carriers:
  Mono/multi composite
* No centers

**FIG. 2M**

Track 1      Track 2      Track 3

Empty Centers

Mono Centers

Multi Carriers
Tracks 2&4

Composite Carriers
Tracks 2&4

Not symm
carriers – less
of these

Track 4

Design 14
* 24 carriers:
  24-end x 60-multifilament
* 12 carriers:
  Mono/multi composite
* No centers

**FIG. 2N**

Track 1      Track 2      Track 3

Empty Centers
Mono Centers
Multi Carriers
Tracks 2&4
Composite Carriers
Tracks 2&4

Track 4

Design 15
- 18 carriers:
  24-end x 60-multifilament
- 18 carriers:
  Mono/multi composite
- No centers

**FIG. 2O**

Track 1      Track 2      Track 3

Empty Centers
Mono Centers
Multi Carriers
Tracks 2&4
Composite Carriers
Tracks 2&4

Track 4

Design 16
- 18 carriers:
  24-end x 60-multifilament
- 18 carriers:
  Mono/multi composite
- 4 centers

**FIG. 2P**

**FIG. 2Q**

32b

30

34

32a

**FIG. 3**

32

**FIG. 4A**

34

**FIG. 4B**

32b

34

32a

36

**FIG. 5A**

32b

34

32a

**FIG. 5B**

**FIG. 6**

**FIG. 7**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62313246
- WO 952550 A
- US 20020123805 A, Murray
- US 20040059416 A
- WO 2007087353 A
- US 20080031923 A, Murray
- US 3797047 A
- US 4187558 A
- US 4483023 A
- US 4610688 A

- US 4792336 A
- US 5061283 A, Silvestrini
- US 5263984 A, Li
- US 8486143 B
- US 8758437 B
- US 20110238179 A, Laurencin
- WO 2009109778 A2
- WO 2004080346 A2
- US 20130302435 A
- US 8563232 B

**Non-patent literature cited in the description**

- **MA et al.** *Nanomedicine*, 2013, vol. 8 (9), 1459-1481
- **DENG et al.** *Trans Nanobiosci*, 2012, vol. 11L3-14
- **WASSERTEIN**. *Sports Health*, 2015, vol. 7 (3), 207-216
- **PALLIS**. *Am J Sports Med.*, June 2020, vol. 40 (6), 1242-1246
- **CHEUNG**. *Knee*, 2012, vol. 19 (1), 49-54
- **DALE et al.** *Acta Orthop*, 2012, vol. 83, 449-458
- **BADYLAK et al.** *Proc Natl Acad Sci USA*, 2010, vol. 107 (8), 3285-3286
- **LANGER** ; **VACANTI**. *Tissue engineering. Science*, 1993, vol. 260 (5110), 920-926

- **KHADEMHOSSEINI et al.** *Proc Natl Acad Sci USA.*, 2006, vol. 103 (8), 2480-2487
- **KHADEMHOSSEINI et al.** *Progress in tissue engineering. Sci Am.*, 2009, vol. 300 (5), 64-71
- **BELLINCAMPI et al.** *J. Orthop. Res.*, 1998, vol. 16, 414-420
- Tendons and Ligaments. **GOULET et al.** Principles of Tissue Engineering. R. G. Landes Company and Academic Press, Inc., 1997, 639-645
- **PISTNER et al.** *Biomaterials*, 1993, vol. 14, 291-298
- **STEVENS**. Polymer Chemistry: an introduction. Oxford University Press, 1999, 10016